# EUROPEAN PATENT APPLICATION

(11) **EP 2 263 580 A2**
(43) Date of publication of application: **22.12.2010**
(21) Application number: 10179010.3
(22) Date of filing: 20.12.2004
(51) Int. Cl.: A61B 17/54, A45D 34/04, A61H 7/00

(54) **Treatment of skin using a benefit agent and an apparatus**

(30) Priority: 24.12.2003 US 746953; 24.12.2003 US 746700; 24.12.2003 US 746701; 24.12.2003 US 746815; 24.12.2003 US 746705; 24.12.2003 US 746446
(62) Divisional of application: 04815020.5
(71) Applicant: Johnson & Johnson Consumer Companies, Inc., Skillman, NJ 08558 (US)
(72) Inventor: Burrows, Mark, Newtown, PA 18940 (US); Cole, Curtis, Ringoes, NJ 08551 (US); Cronin, John, Essex Junction, VT 05452 (US); Gubernick, David, Cherry Hill, NJ 08003 (US); Hull, Raymond, Hampton, NJ 08827 (US); Menke, James, Califon, NJ 07830 (US); Narsana, Tushar, Essex Junction, VT 05452 (US); Rytel, John F., East Brunswick, NJ 08816 (US); Skover, Gregory, Princeton, NJ 08540 (US)
(74) Representative: Tunstall, Christopher Stephen

(57) **Abstract**

The invention relates to an apparatus for delivering mechanical energy to an expanse of skin, said apparatus comprising: a skin-contactable element having a skin-contactable surface comprising a first and second sub-surface, wherein said skin-contactable element comprises an agent selected from the group consisting of a benefit agent, an apparatus-enhancing agent, and combinations thereof; a motor; and a transfer member for transferring mechanical energy from said motor to said skin-contactable element in order to provide periodic motion to said skin-contactable surface; wherein said periodic motion comprises a first periodic motion of said first sub-surface and a second periodic motion of said second subsurface such that said second subsurface moves in a disjoint relationship with respect to said first sub-surface.

## Description

### FIELD OF THE INVENTION

The present invention relates to treating the skin of a subject, and, in particular, to treating the skin of a subject using mechanical energy.

### BACKGROUND OF THE INVENTION

With advances in nutrition and medical treatment, the life expectancy of the average U.S. and world citizen has increased dramatically. As a result, large portions of those populations suffer from the associated effects of aging, including an increasing number of skin health issues. Though seldom life threatening, skin health issues can be uncomfortable and may cause chronic disabilities. In addition, because the skin is so visible, skin health issues and cosmetic skin conditions can lead to psychological stress in the patients who have them. Many members of the aging population have also become increasingly educated regarding general physical health and ways of looking and feeling better about physical appearance. This desire for good health and appearance has driven people to seek improved solutions to health care and skin care.

Numerous techniques have been proposed to provide cosmetic and/or or skin rejuvenation benefits. One of the more popular, professional microderm abrasion, is a non-invasive procedure in which a device pulls the skin via suction and bombards the skin with abrasive particles in order to affect an exfoliation. Professional microderm abrasion devices, however, are cumbersome in that they occupy a large amount of space and also require a high power input and must be plugged into an AC outlet during operation. Furthermore, the patient must make regular visits to the professional skin care specialist where he or she receives treatment. This is inconvenient and may be expensive. Furthermore, they tend to be messy and embed the particles into the skin may be difficult to remove. They also may heat the skin to an uncomfortable temperature and cause excessive irritation to the skin.

The present invention relates to a device that imparts benefits to the skin without some or all of the drawbacks of professional microderm abrasion.

### SUMMARY OF THE INVENTION

In one aspect, the invention features a method of administering a skin benefit agent to an expanse of skin, wherein the method includes: (a) contacting the expanse of skin with a skin benefit agent; and (b) contacting the skin benefit agent on the expanse of skin with an apparatus having a output power to the skin of greater than about 0.2W, the apparatus including: a skin-contactable element having a skin-contactable surface; a motor; and a transfer member for transferring mechanical energy from the motor to the skin-contactable element in order to provide periodic motion to the skin-contactable surface; wherein the skin-contactable surface contacts the skin-benefit agent.

In another aspect, the invention features a method of administering a skin benefit agent to an expanse of skin, wherein the method includes: (a) contacting the expanse of skin with an apparatus, the apparatus including:(i) a skin-contactable element having a skin-contactable surface; (ii) a motor; and (iii) a transfer member for transferring mechanical energy from the motor to the skin-contactable element in order to provide periodic motion to the skin-contactable surface; and (b) after contacting the expanse of skin with the apparatus has ceased, further contacting the expanse of skin with a benefit agent, wherein the benefit agent is selected from the group consisting of retinoids, copper moieties, skin-firming agents, depigmentation agents, and combinations thereof.

In another aspect, the invention features a product that includes a composition containing a skin benefit agent selected from the group consisting of retinoids, copper moieties, skin-firming agents, depigmentation agents, and combinations thereof, wherein the product further includes instructions directing the user to apply the composition to an expanse of skin following contact of the expanse of skin with an apparatus that imparted mechanical energy to the expanse of skin.

In another aspect, the invention features an apparatus for delivering mechanical energy to an expanse of skin, wherein the apparatus includes: (a) a skin-contactable element having a skin-contactable surface, wherein the skin-contactable element includes an agent selected from the group consisting of a benefit agent, an apparatus-enhancing agent, and combinations thereof; (b) a motor; and (c) a transfer member for transferring mechanical energy from the motor to the skin-contactable element in order to provide periodic motion to the skin-contactable surface; wherein the agent is configured to release from the skin contactable element to the expanse of skin upon contacting the skin-contactable surface with the expanse of skin.

In another aspect, the invention features a product includes: (a) a skin-contactable element having a skin-contactable surface, wherein the skin-contactable element includes an agent selected from the group consisting of a benefit agent, an apparatus-enhancing agent, and combinations thererof; and (b) instructions directing a user to couple the skin-contactable element to a motorized apparatus, wherein the motorized apparatus is for imparting mechanical energy to the skin-contactable surface; wherein the agent is configured to release from the skin contactable element to the expanse of skin upon contacting the skin-contactable surface with the expanse of skin.

In another aspect, the invention features an apparatus for delivering mechanical energy to an expanse of skin, wherein the apparatus includes: (a) a skin-contactable element having a skin contactable surface; (b) a motor; and (c) a transfer member for transferring mechanical energy from the motor to the skin-contactable element in order to provide periodic motion to the skin-contactable surface; wherein the skin-contactable element includes a signaling marker that is adapted to provide a change in a sensation that is discernable to a user after a period of time of operation of the apparatus, and wherein the sensation is selected from a group consisting of tactile, olfactory, thermal, visual, auditory, and combinations thereof.

In another aspect, the invention features a product includes: (a) a skin-contactable element including a signaling marker that is adapted to provide a change in a sensation that is discernable to a user after a period of time of operation of the apparatus, and wherein the sensation is selected from a group consisting of tactile, olfactory, thermal, visual, auditory, and combinations thereof; and (b) instructions directing a user to couple the skin-contactable element to a motorized apparatus, wherein the motorized apparatus is for imparting mechanical energy to the skin-contactable surface.

In another aspect, the invention features an apparatus for delivering mechanical energy to skin, wherein the apparatus includes: (a) a user output assembly, the user output assembly including: (i) a skin-contactable element having a skin contactable surface; (ii) a motor; and (iii) a transfer member for transferring energy from the motor to the skin-contactable element a transfer member for transferring energy from the motor to the skin-contactable element; (b) a receiving element adapted to receive user-attribute data that is provided to the apparatus by a user; and (c) a controller coupled to the receiving element and the user output assembly, wherein the controller provides action instructions to the user output assembly based upon the user-attribute data received by the receiving element.

In a further aspect, the invention features a method for delivering mechanical energy to skin, wherein the method includes: (a) providing user-attribute data to the receiving element of the apparatus described in the paragraph immediately above; and (b) contacting an expanse of skin with a skin-contactable surface of the apparatus.

In another aspect, the invention features an apparatus for delivering mechanical energy to an expanse of skin, wherein the apparatus includes:(a) a user output assembly, the user output assembly including:(i) a skin-contactable element having a skin contactable surface; (ii) a motor; and (iii) a transfer member for transferring energy from the motor to the skin-contactable element a transfer member for transferring energy from the motor to the skin-contactable element; (b) a sensing element in communication with the skin-contactable surface, wherein the sensing element is capable of sensing a state of at least one property associated with the expanse of skin, wherein the at least one property is selected from the group consisting of a thermal property, a chemical property, an optical property, and combinations thereof; and (c) a controller coupled to the sensing element and to the user output assembly, wherein the controller provides action instructions to the user output assembly based upon one or more of the at least one property sensed by the sensing element.

In a further aspect, the invention features a method for delivering mechanical energy to skin, wherein the method includes contacting an expanse of skin with a skin-contactable surface of the apparatus described in the paragraph immediately above.

Other features and advantages of the present invention will be apparent from the detailed description of the invention and from the claims.

### BRIEF DESCRIPTION OF THE DRAWINGS

A more particular description of the invention, briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.

Figure 1 is a cross-sectional, schematic view of various components of a skin treatment system that is consistent with embodiments of the invention described herein.

Figure 2A is a top view of a mechanical energy delivery sub-assembly that may be used in an apparatus consistent with embodiments of the invention described herein.

Figure 2B is a side view of the mechanical energy delivery sub-assembly of Figure 2A.

Figure 3A is a fragmented, 3-dimensional top perspective view of a skin-contactable element being placed in association with a transfer member in a manner consistent with embodiments of the invention described herein.

Figure 3B is a fragmented, 3-dimensional top perspective view of an alternative embodiment of a skin-contactable element being placed in association with a transfer member in a manner consistent with embodiments of the invention described herein.

Figure 4A-4F are fragmented side views of an apparatus consistent with embodiments of the invention described herein, depicting various forms of motion that may be imparted to an expanse of skin in contact therewith.

Figure 5A is a top view of another embodiment of a mechanical energy delivery sub-assembly that may be used in a manner consistent with embodiments of the invention described herein.

Figure 5B is a side view of the mechanical energy delivery sub-assembly of Figure 5A.

Figure 6A-6E are three dimensional top perspective views of skin-contactable elements that may be used for contacting the skin according to embodiments of the invention described herein.

Figure 7A is a side view of a test apparatus useful for determining the power output of a mechanical device to skin.

Figure 7B is a side view of a test apparatus of Figure 7A, adapted to determine a baseline power output of a mechanical device to skin.

Figure 8 is a fragmented, cross-sectional view of a skin-contactable element having a multi-layer structure, consistent with embodiments of the invention described herein.

Figure 9 is a block diagram of a controller suitable for use with embodiments of the invention described herein.

Figure 10A-D depict examples of waveforms that may be generated by an apparatus consistent with embodiments of the invention described herein.

To facilitate understanding identical reference elements have been used, wherever possible, to designate identical elements that are common to the Figures.

### DETAILED DESCRIPTION OF THE INVENTION

Unless defined otherwise, all technical and scientific terms used herein have the same meaning as commonly understood by one of ordinary skill in the art to which the invention belongs. Also, all publications, patent applications, patents, and other references mentioned herein are incorporated by reference.

### SKIN TREATMENT SYSTEM

Embodiments of the invention described herein relate to treatment of the skin using mechanical energy. In general, mechanical energy may be provided to skin using an apparatus for delivering mechanical energy to the skin, and an optional diagnostic sub-system that may be used to perform diagnostic assessment of the skin to be treated. The apparatus generally includes (1) a user output system for providing mechanical energy, (2) an optional controller for processing information and providing instructions to the user output system, (3) an optional receiving element for receiving certain forms of data and providing it to the controller, (4) an optional sensing element for providing additional data to the controller, and (5) an optional energy storage element for providing energy to the user output system. Those components (1) through (5) discussed above that are present in the skin treatment system are desirably, but not essentially housed within a single, integrated hand-held unit.The user output system generally includes (1) a mechanical delivery sub-assembly for transmitting and delivering mechanical energy to the skin and (2) an optional chemical delivery subassembly for delivering various fluid compositions to the skin.

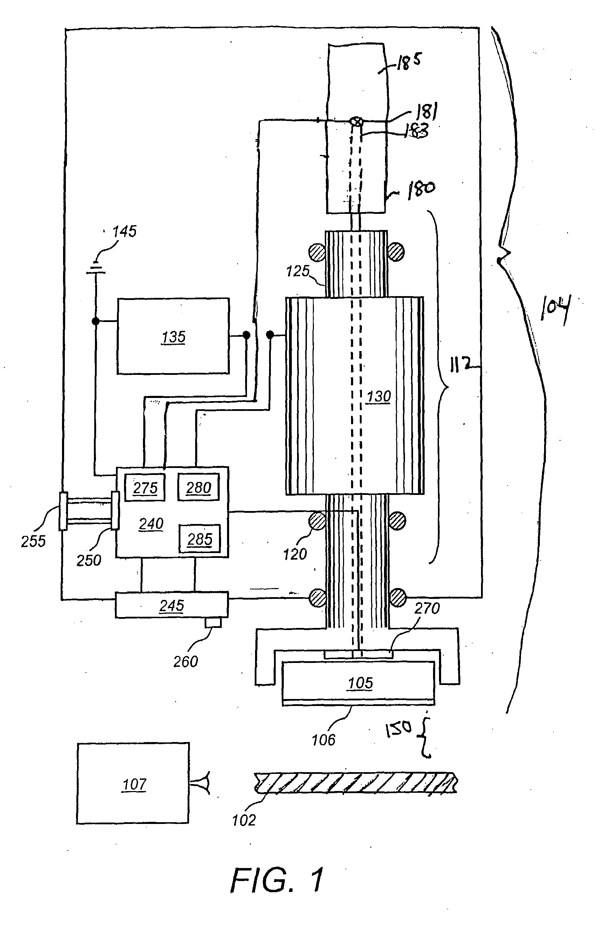

Figure 1 depicts an apparatus 100 for treating an expanse of skin 102 of a subject (*e.g*., a mammalian subject such as a human). The expanse of skin 102 may be restricted to the face or may include one or more other regions of the body, such as, for example legs, arms, buttocks, neck, back, nails, among other bodily locations. By "treating", it is meant that one or more of the following benefits (through treatment or prevention via various biochemical and/or mechanical mechanisms) are imparted to the subject: skin rejuvenation benefits such as younger, healthier, radiant skin, even or non-blotchy texture tone and/or texture, removal or reduction of the appearance of such features as wrinkles or fine lines, surface roughness, folds or sagging, surface vessels, age spots/pigmentation, redness, scars from acne or other sources, and pore size; body re-shaping benefits such as removal/ reduction of cellulite or body fat; treatment/reduction of acne lesions and/or pain associated therewith; hair-growth stimulation; and hair growth retardation or removal.

The apparatus 100 includes a user output assembly 104 whose general function is to deliver mechanical energy and optionally deliver various agents to the expanse of skin 102 of the subject. By "agents" it is meant either "benefit agents" or "apparatus-enhancing agents," as described, respectively, in this specification with reference to the sections below entitled, "BENEFIT AGENTS" and entitled "APPARATUS-ENHANCING AGENTS." The user output assembly 104 includes a mechanical energy delivery sub-assembly 112, which includes a motor 130, for converting stored energy from an energy storage element 135, and a transfer member 125 for transferring energy from the motor 130 to a skin-contactable surface 106. The user output assembly 104 may also include a chemical delivery sub-assembly 180, such as for delivering fluids that may include various agents to the expanse of skin 102. The user output assembly 104 may also include an indicator 245 to facilitate communication between the apparatus 100 and at least one operator of the apparatus 100 such as by providing visual, audible or tactile stimulation to the operator. Note that apparatus 100 may be operated solely by the subject, in which case the operator and subject are identical. Alternatively one or more separate operators (*e.g*., doctors, technicians) may operate the apparatus 100 to provide skin-treatment benefits for the subject. Note that the term "user" as discussed in this document refers to either or both of the subject or the operator.

The apparatus 100 generally includes an input 260 such as one or more buttons or switches that enable the operator to turn the apparatus 100 on and off, and may further customize the control of the motion of skin-contactable surface 106. The apparatus may also include a controller 240 for providing instructions to the user output assembly 104.

In one embodiment of the invention, the apparatus 100 further includes a receiving element 255 coupled to the controller 240. The receiving element 255 is capable of receiving data such as user-attribute data (*e.g*., height, weight, age, and/or body mass index). In another embodiment of the invention, the apparatus 100 includes a sensing element 270 coupled to the skin-contactable surface 106. The sensing element 270 is capable of sensing at least one state of each of one or more properties associated with the skin. The sensing element 270 is, for example, electrically coupled to the controller 240. The controller 240 may provide action instructions to the user output assembly 104, the instructions based upon one or more properties sensed by the sensing element 240.

Energy may be provided to the motor 130, using, for example, the energy storage element 135 shown in Figure 1 (*e*.*g*. a DC power source such as a battery) coupled to the motor 130 and grounded using a power supply ground 145. Alternatively, the motor 130 may be coupled to power source that is external to the apparatus 100, such as an AC source (*e.g*. a 110 or 220 volt wall socket receptacle).

The apparatus 100 may be a part of a system 1 for treating the skin. In this embodiment of the invention, the system 1 includes the apparatus 100 and a diagnostic sub-system 107 for diagnosing various conditions or states of the skin 102 (e.g., by measuring components of the blood or interstitial fluid on the skin, by imaging the surface of the skin, or by measuring UV reflectance of the skin).

### MECHANICAL ENERGY DELIVERY SUB-ASSEMBLY

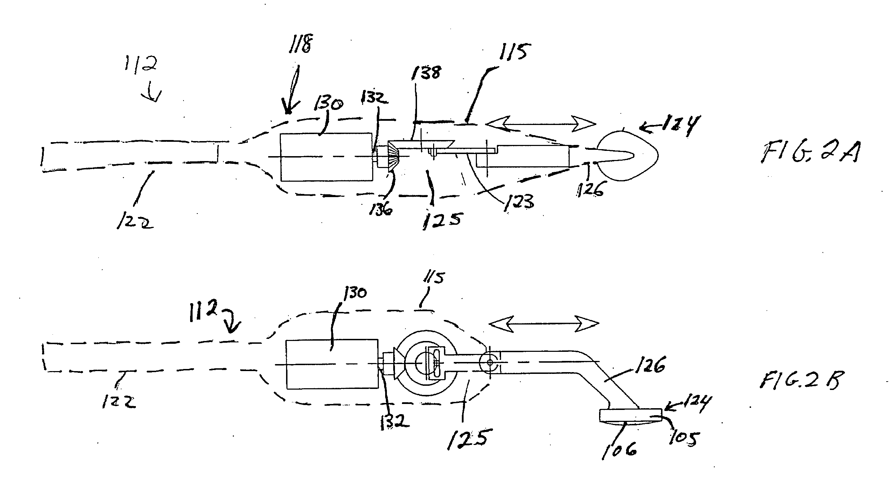

The user output assembly 104 of the apparatus 100 generally includes mechanical energy delivery sub-assembly 112, an example of which is shown in Figures 2A-2B, for providing mechanical energy to the expanse of the skin 102 of the subject. Figures 2A and 2B depict a top view and a side view, respectively, of the exemplary mechanical energy delivery sub-assembly 112 that includes the motor 130 for converting electrical energy that is provided to the motor 130 into mechanical energy for actuating the transfer member 125. The transfer member 125 includes, in this embodiment of the invention, a driver shaft 132 that is coupled to the motor 130 and rotated thereby. The transfer member 125 may further include other mechanical elements for transmitting motion from the motor to the skin-contactable surface 106 (the arrows in Figures 2A-B depict one example of back and forth or "reciprocating" motion that may be imparted to the skin-contactable surface 106). For example, in this embodiment of the invention, transfer member 125 further includes a slotted cam arm 123 that is coupled to the driver shaft 132 via a driver bevel gear 136 and a cam bevel gear 138.

The apparatus 100 may be completely or partially encased or shrouded in a shell 115 (shown in phantom in Figures 2A and 2B) that houses various components (*e*.*g*., the motor 130, the transfer member 125) of the apparatus 100. The shell 115 is generally for protecting these components from environmental challenges and to facilitate portability of the apparatus 100. The shape defined by the shell 115 varies, but the shape thus defined is generally such that the apparatus 100 is readily grasped by the user such that the skin-contactable surface 106 may be positioned in contact with the expanse of skin 102. The apparatus 100 may include, for example, a generally linear main body region 118 that includes a handle 122. The apparatus 100 may include a head region 124 that includes the skin-contactable surface 106 (shown in Figure 2B). Referring to Figure 1, the mechanical energy delivery sub-assembly 112 may also include various mechanical parts such as bushings 120 to reduce friction, thereby permitting the device to deliver a high percentage of power that is provided by the energy storage element 135 to the skin-contactable surface 106.

Referring to Figures 2A and 2B, the main body region 128 and the head region 124 may be connected by a connecting region 126 that may be angled to enhance both the ability of the user to grasp the apparatus 100 and position the skin-contactable surface 106 against the expanse of skin 102 and move the apparatus 100 across the expanse of skin 102.

### SKIN CONTACTING SURFACE

The skin-contactable surface 106 may be, for example, a surface (*e.g*. an outer surface) of a skin-contactable element 105 that is coupled to the transfer member 125, and may be shaped to facilitate contact with the expanse of skin 102. The skin-contactable element 105 may be removable, replaceable or attachable/detachable, such as, for example, a unitary module or cartridge. The skin-contactable element 105 may have a high degree and/or predictable resistance to chemicals or compositions that may contact the skin-contactable element 105 during use. Furthermore, the skin-contactable element 105 may have a high degree and/or predictable mechanical durability. The detachability of the skin-contactable element 105 facilitates removal of the skin-contactable element 105 when it is old, worn, microbiologically contaminated or spent, such that it may be replaced with a new or fresh skin-contactable element 105. The element 105 may be, for example, discarded after a single use.

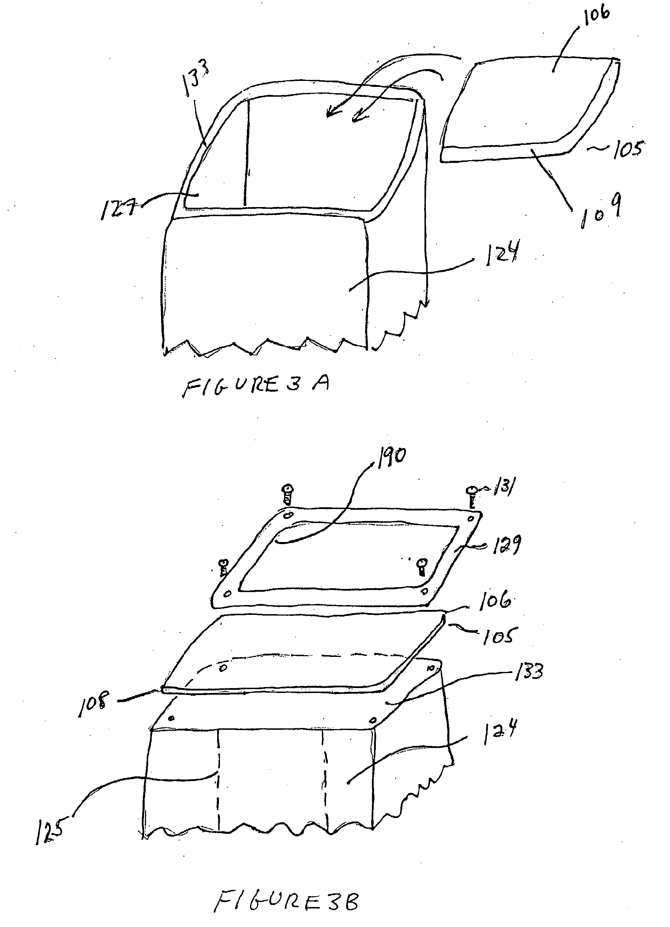

Figure 3A depicts one embodiment of the invention in which the skin contactable element 105 is a pad that is placed in association with the transfer member 125 (transfer member 125 is shown in Figure 1). The skin-contactable element may be mated to the skin contactable element 105 to a recess 127 within the head region 124 for accepting the skin contactable element 105. The skin-contactable element 105 may include a securing surface 109 for contacting a mating surface 133 of an apparatus so as to temporarily secure the skin-contactable element to the mating surface 133 during operation of the apparatus. The securing surface 109 may mate with the mating surface 133 by various mechanisms, one of which is shown in Figure 3A. In order to facilitate securing of the skin-contactable element 105, the securing surface 109 and/or the mating surface 133 may be slightly flexible, yet resilient to permit (1) easy mating of the surfaces 109, 133; (2) firm securement during operation; as well as (3) easy removability, to facilitate replacement of the skin-contactable element 105 when required.

Alternatively, the skin contactable element 105 may be held in place during operation of the apparatus 100 via any number of suitable mechanical or magnetic components (not shown), such as clamps, snaps, adhesive, and the like may be used to facilitate attachability and detachability of the skin-contactable element 105.

The skin contactable surface 106 of the skin contactable element 105 may be designed such that an interface 150 (see Figure 1) between the skin contactable surface 106 and the expanse of skin 102 has sufficient friction to couple energy with minimal loss into the expanse of skin 102.

Figure 3B depicts another embodiment of the invention in which the skin contactable element 105 is or includes a porous material, such as a porous sheet 108 (*e.g*. a free-standing sheet that is readily detachable from the apparatus 100). The pores may be capable of transporting liquid (*e.g*., being or containing a "benefit agent," as defined below in the section entitled "BENEFIT AGENTS") from within the skin-contactable element 105 to the skin-contactable surface 106 of the skin-contactable element 105. The sheet 108 may be fibrous and/or film-based (*e.g*., may include fibrous and/or plastic film materials, such as one or more layers of these materials). The layers of material may be relatively rigid or relatively compliant and may serve one or more functions such as enhancement of friction at the interface 150, transport of sebum away from the skin, transport of various agents towards the skin so that they may provide some benefit thereto, among other functions.

Suitable fibrous materials that may be used include those based from organic polymers such as, for example, polyester, polyolefin, rayon, cellulose such as from wood pulp, bicomponent fibers, fibers coated with antimicrobial agents such as zinc, silver, or copper (*e.g*., silver-coated polymer fibers commercially available from Noble Fiber Technologies of Clarks Summit, Pennsylvania) and other combinations thereof. The fibers are woven or non-woven and arranged in a network via, for example, a carding process, and bonded via, for example, an air-through bonding, chemical bonding, or an embossing process. The layer of fibrous material may include binders such as organic resins or other ingredients to manipulate the mechanical or fluid management properties thereof. The layer of fibrous material may have a basis weight that supports the layer to maintain its mechanical integrity for one or more uses of the skin contactable element 105. The basis weight may be, for example, between about 10 grams per square meter (gsm) and about 100 gsm, such as between about 40 gsm and about 60 gsm.

The layer of fibrous material may include fibers that are not so soft as to promote the sheet from readily slipping across the expanse of skin 102. For example, the fibers may be free of softening or conditioning additives such as coatings of conformal polymers or coatings of cationic or other surfactants. In one embodiment of the invention, the fibers have a denier between about 1 denier per fiber (dpf) and about 30 dpf. A denier of between about 3 dpf and about 6 dpf may be suitable to enhance the friction at the interface 150. The layer of fibrous material may be thick and/or lofty (having considerable amount of "fluff' or void space between the fibers). Such layers may enhance comfort to the user and/or facilitate easy contact with the expanse of skin 102.

Suitable plastic films that may be used in the sheet 108 include apertured thermoplastic films such as those comprising polyethylene, polypropylene or similar materials. The apertured film may be designed to enhance friction at the interface 150, to manage the movement of agents, or otherwise create a suitable skin-contactable surface 106. The thickness of the apertured thermoplastic film may be in a range from about 0.5 mm. to about 1 mm before aperturing. The apertures may be oriented towards or away from the expanse of skin 102. In one embodiment of the invention, the thermoplastic apertured film has a specific gravity in a range from about 0.04 grams per cubic centimeter (g/cc) to about 0.12 g/cc. The apertures may be present in a number density from about 10 apertures per square centimeter to about 100 apertures per square centimeter.

The porous sheet 108 may be secured to the transfer member 125 (shown in phantom in Figure 3B) via any number of methods, but the method of securing generally is chosen such that bunching and folding of the sheet 108 during operation of the apparatus 100 are minimized or eliminated. Figure 3B depicts one suitable way in which the sheet is coupled to the transfer member 125 by urging the sheet 108 against mating surface 133, placing a frame 129 against the sheet 108, and using mechanical fasteners 131 (*e.g*. screws, clamps, clips, clasps, etc. that may slide onto, pinch, or penetrate through the sheet 108) to secure the sheet 108 to the head region 104 of the apparatus 100. The frame 129 has a geometry that is selected such that it edges 190 generally are not sharp, so as to prevent pinching or cutting the expanse of skin 102 (*e*.*g*., edges 190 may be beveled or slope downward towards the mating surface 133). The frame 129 may be formed from materials that are resistant to corrosion, such as might otherwise occur from being exposed to various fluids used in conjunction with the apparatus 100.

The mating surface 133 shown in Figure 3B serves as only one general example for securing the sheet to the transfer member 125. Alternatively, or in addition, other methods may be used to secure and detach the sheet 108 and/or to prevent the sheet 108 from bunching or folding during use. For example, micro hooks, adhesive, and the like may be used for this purpose.

### ACTUATION OF SKIN-CONTACTABLE SURFACE

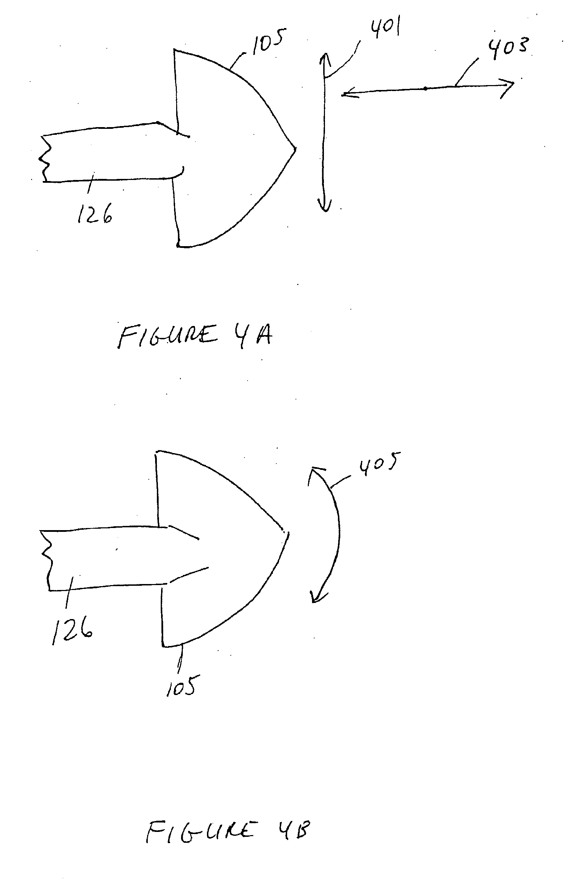

Referring to Figures 1-4, the transfer member 125 transfers mechanical energy from the motor 130 to the skin-contactable surface 106. The skin-contactable surface 106 may be thereby urged to actuate in a periodic fashion so as to impart energy to the skin 102. By "periodic," it is meant that the skin-contactable surface 106 moves cyclically, in a fixed or random direction, with a frequency greater than about 1 cycle per second. The motor 130 may be a conventional motor that is capable of imparting periodic motion to the transfer member 125 that is coupled to the motor 130 so that the transfer member 125 moves in one or more directions. For example, the transfer member 125 may move such that the motion of the skin contactable surface 106 is essentially planar, *i.e*., the movement of the skin contactable surface 106 is adapted to be essentially parallel to the expanse of skin 102. This may be accomplished, for example, by having the skin-contactable surface 106 move with reciprocating motion (*i.e.,* the skin-contactable surface repeatedly retraces a path that is essentially only a linear or curved segment, examples of which are shown in Figures 4A-B, with arrows 401, 403, 405 indicating examples of suitable paths of motion).

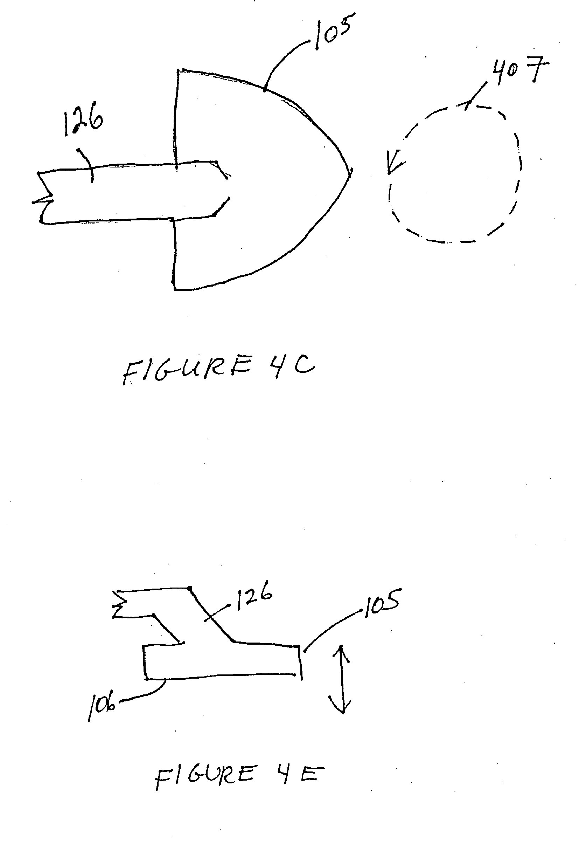

In another embodiment of the invention, the skin-contactable surface 106 moves with orbital motion. By "orbital motion", it is meant that the skin-contactable surface 106 travels a path that is open, (*e.g*., elliptical, circular, polygonal, star-shaped, or multisegmented), an example of which is shown in Figure 4C, with arrow 407 indicating an exemplary path of motion.

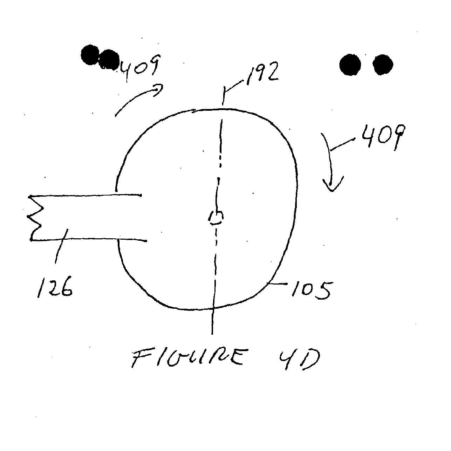

In another embodiment of the invention as shown in Figure 4D, the skin-contactable surface 106 is adapted to produce pure rotational motion about a central axis 192 (the axis 192 is shown in phantom in Figure 4D and is perpendicular to the plane of the paper, and arrows 409 indicate exemplary rotation within the plane of the paper)

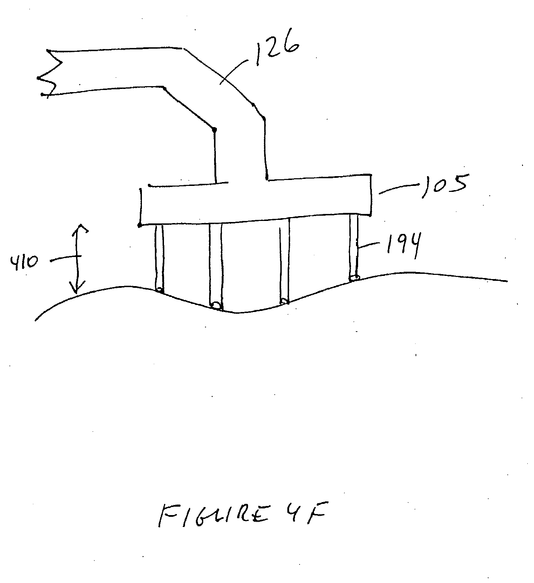

As shown in Figure 4E-F (side views), in another embodiment of the invention, the skin-contactable surface 106 is adapted to move in a periodic fashion that is substantially perpendicular (*e.g*., substantially normal to) the expanse of skin 102, as shown by arrows 410, to produce a "tapping" or "thumping" motion against the expanse of skin 102. Figure 4F depicts the skin-contactable surface 106 that includes a plurality of independent subsurfaces 194. In this embodiment of the invention, the independent subsurfaces 194 each provide independent motion perpendicular to the expanse of skin 102. The independent subsurfaces 194 may be, for example, "fingers" that are, for example, naturally extended via springs that may at times be become compressed depending upon the curvature of the expanse of skin 102 and the position in which the independent subsurfaces 194 are placed thereon. Alternatively, the subsurfaces 194 may be motor-driven to tap simultaneously or sequentially.

The motor 130 may be optimized to produce any one of these motions depicted in Figures 4A-4F or several combinations of forms of motions (*e.g*., reciprocating, rotational, orbital, tapping, and combinations thereof). These various combinations of forms of motion may be particular advantageous to, for example, impart benefits to the expanse of skin 102 by stretching, massaging, kneading or otherwise stimulating the expanse of skin 102. This may be particularly useful for promoting enhanced circulation of blood or lymphatic fluid in the tissues that are proximate the expanse of skin 102. As such, various benefits may result, including but not limited to, skin rejuvenation benefits.

The skin-contactable surface 106 may be adapted to move with periodic motion that has a frequency that is in a range of about 1500 cycles per minute to about 5,000 cycles per minute. The skin-contactable surface 106 may move with an amplitude (i. e., the greatest linear distance that any point on the skin- contactable surface 106 moves between during the course of a cycle of its periodic motion) that is in a range of about 0.5 millimeters (mm) to about 10 mm, such as from about 1mm to about 5mm, such as between about 2mm to about 3 mm.

Note, while the mechanical energy delivery sub-assembly 112 is described above as imparting periodic motion to the skin contactable surface 106, alternatively, the mechanical energy delivery sub-assembly 112 may be designed to impart mechanical energy that is not necessarily periodic. For example, the mechanical energy delivery sub-assembly 112 may be designed to "suck" the expanse of skin 102 such as by coupling a vacuum to the skin-contactable surface 106 (*e.g*. in this embodiment of the invention, the skin-contactable surface 106 may have an annular-shape). In another embodiment of the invention, the mechanical energy delivery sub-assembly 112 may include rollers or other devices designed to knead, pluck, pinch, or glide against the skin to impart energy thereto. An exemplary device that may be used to impart one or more of these alternative forms of energy to the skin is described in U.S. patent number 6,017,320 and European Patent No. 1,045,685B1.

### SUB-SURFACES MOVING IN DISJOINT RELATIONSHIP

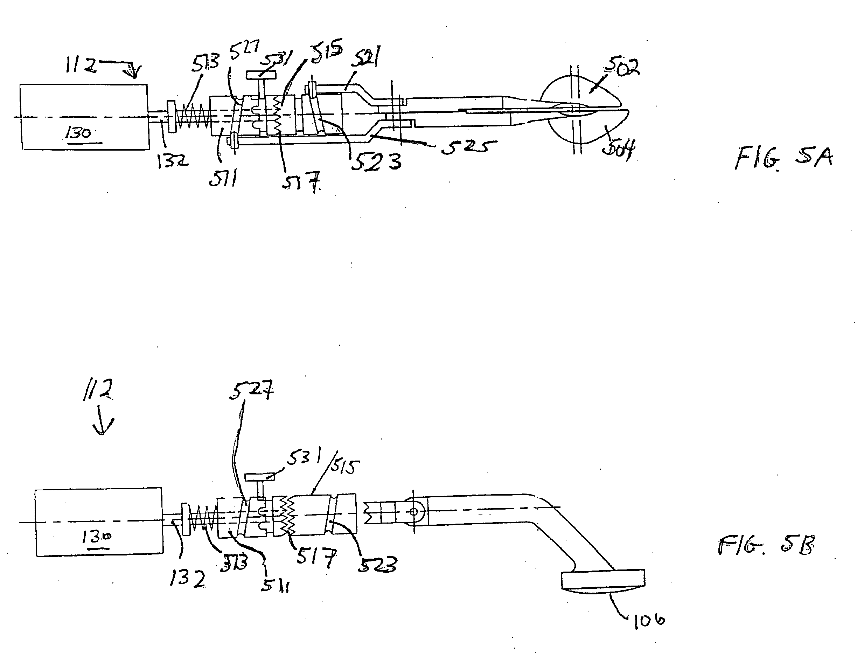

Figures 5A and 5B depict top views of another embodiment of the mechanical energy delivery sub-assembly 112 in which the skin-contactable surface 106 includes a first sub-surface 502 and a second subsurface 504. The second subsurface 504 is adapted to move in a disjoint relationship with the first sub-surface 502. By "disjoint relationship", it is meant that the first sub-surface 502 and the second subsurface 504 each move periodically and share a same type (*e.g*., vibration, rotation, orbital, tapping) of motion, but are capable of movement in an opposite sense (e.g., forward as opposed to backward or clockwise as opposed to counterclockwise). Stated in other words, when the first sub-surface 502 has in moving in one direction, the second subsurface 404 may be moving in the opposite direction.

According to one exemplary embodiment of the invention, depicted in Figures 5A-5B, the motor 130 is rotatably coupled to the driver shaft 132. The driver shaft 132 is coupled to a slide tooth barrel cam 511, which is in turn is coupled to a fixed tooth clutch barrel cam 515 via a mating set of teeth 517 that are urged together by a spring 513.

The fixed tooth clutch barrel cam 515 transfers rotational motion from the shaft 132 into, for example, reciprocating linear motion that is imparted to a first driver arm 521. The transfer of rotational motion may be accomplished by, for example, fixing one end of the first driver arm 521 into alignment with a first slanted groove 523 that circumscribes the cam 515. The first driver arm 521 is coupled to the first sub-surface 502, imparting a first periodic motion thereto.

Similarly, the slide tooth barrel cam 511 transfers rotational motion from the shaft 132 into reciprocating linear motion that is imparted to a second driver arm 525. The transfer of rotational motion may be accomplished again by fixing one end of the second driver arm 525 into alignment with a second slanted groove 527 that circumscribes the cam 511. The second driver arm 525 is coupled to the second sub-surface 504, imparting a second periodic motion thereto.

While the first subsurface 502 and the second sub-surface 504 may move with periodic motion having the same or similar amplitude, this is not required. Furthermore, a variable phase difference may be imparted between the first periodic motion of the first sub-surface 502 and second periodic motion the second sub-surface 504 by, for example, adjusting the position of a pin 531 to compress the spring 513, thereby disengaging the teeth 517. Upon disengagement of the teeth 517, the cams 511, 517 may be rotated with respect to one another, and thereafter re-engaging the teeth 517. After such adjustment, the relative position of the first driver arm 521 and the second driver arm 525 is thereby set such that a phase difference that is non-zero between the motion of the drivers arms 521, 525 will be achieved upon providing motion to the shaft 132.

In one embodiment of the invention, the motion of is set to be out of phase by about 180 degrees (*i*.*e*., the velocity of the first sub-surface 502 and the second subsurface 504 is essentially at any particular moment in time never in the same sense). This phase difference may be suitable to deliver energy to the expanse of skin 102 that is concentrated near the outer surface of the expanse of skin 102 (*i*.*e*., that portion of the expanse of skin 102 that is proximate to the skin-contactable surface 106). In another embodiment of the invention, the motion is set to be substantially "in-phase" (*i.e*., the motion of the first sub-surface 502 and the second subsurface 504 is out of phase by about 0 degrees). This setting may suitable to deliver energy to the expanse of skin 102 that is concentrated deeper within the expanse of skin 102 (*e.g*., deeper layers of the dermis). Of course, the motion may be set anywhere in between 0 degrees and 180 degrees to "tune" or customize either (1) the depth within the expanse of skin 102 to which the energy is delivered or (2) the degree of stretching or twisting motion imparted to the expanse of skin 102.

While the linear motion depicted in Figures 5A and 5B represent one way that the first sub-surface 502 and the second subsurface 504 may move in a disjoint relationship, other motion that is characterized as having a disjoint relationship is contemplated. For example, the first sub-surface 402 and the second subsurface 404 may rotate about different axes of rotation where the sense of rotation of the first sub-surface 402 and the second subsurface 404 is opposite and therefore disjoint. In another embodiment of the invention, the individual subsurfaces 194 shown in Figure 4F may be motor driven to tap in a disjoint relationship with one another.

### SKIN-CONTABLE SURFACE AND FRICTION-ENHANCEMENT

In one embodiment of the invention, the skin-contactable surface 106 is adapted to couple to the expanse of skin 102 along an interface 150 (as shown in Figure 1) that is friction-enhanced. In this manner, the amount of energy that is delivered to the expanse of skin 102 is accentuated. The friction-enhancement of the interface 150 may be accomplished through various manners.

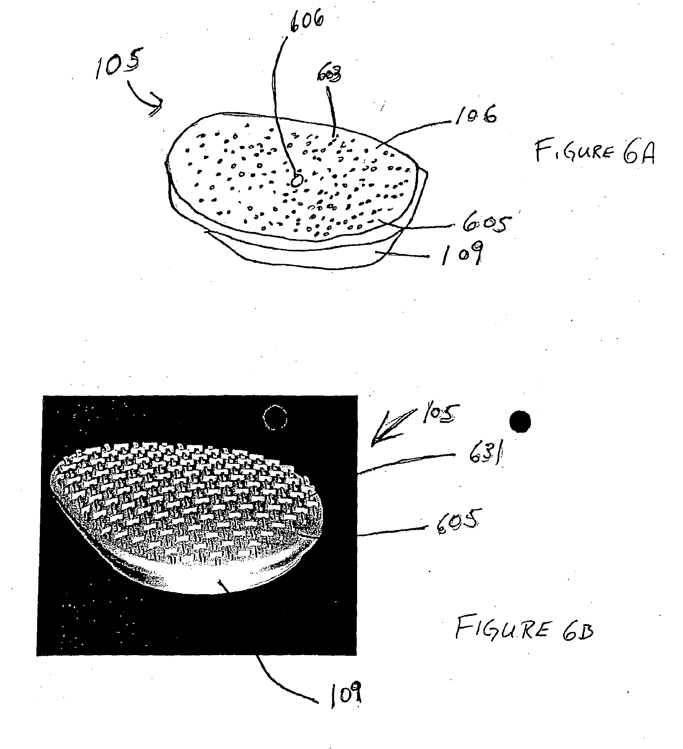

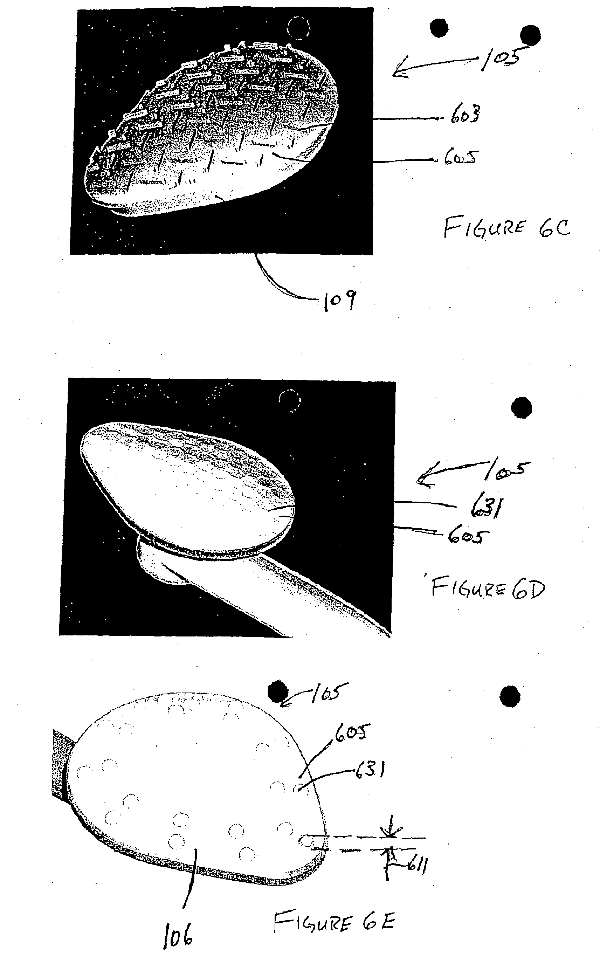

As shown in Figure 6A, in one embodiment of the invention, the skin-contactable surface 106 includes raised, non-cutting regions 603 protruding from a primary surface 605. The raised, non-cutting regions 603 are generally at least partially affixed to a primary surface 605 as shown in Figure 6A. By "at least partially affixed" it is meant that the raised, non-cutting regions 603 are permanently affixed or, alternatively, affixed in a manner such that the particles 605 are readily releasable from the primary surface 605. The primary surface 605 may be contoured to facilitate contact with the expanse of skin 102 (shown in Figure 1). The primary surface 605 of the skin-contactable surface 106 may be arcuate and concave as shown in Figure 6A-6F, or the skin-contactable surface 106 may be portions that are planar or convex.

The raised, non-cutting regions 603 may protrude from the primary surface 605 to such a degree such that the primary surface 605 does not readily contact the expanse of skin 102 of the subject. Alternatively, the raised, non-cutting regions may be relatively small such that they protrudes from the primary surface 605 only slightly, such that the primary surface 605 is capable of contacting the skin 102 of the subject (and is therefore part of the skin-contactable surface 106). The raised, non-cutting regions 603 may be curved and smooth rather than sharp and angular like conventional abrasive exfoliating particles (ground walnut shells, ground apricot shells, ground inorganic particles, and the like). Smooth and curved and/or non-cutting and/or polished friction-enhancing particles 603 are advantageous in that such raised, non-cutting regions 603 are capable of enhancing friction at the interface 150, but the skin 102 is not subject to microcutting, scratching or undue abrasion from contact with the raised, non-cutting regions 603. The raised, non cutting regions 603 are believed to assist in the removal of dead skin cells from the surface, but damage the attached living surface is mitigated.

One example of suitable non-cutting particles that may be included in the raised, non-cutting regions 603 are, for example, particles of polished glass, such as silica-based particles. The particles may have a particle size that is less than about 100 microns, such as in a range from about 25 microns to about 100 microns. The particles may be attached to the primary surface 605 by various means, such as by applying an epoxy or other adhesive to a plastic or elastomeric substrate that has primary surface 605 and then allowing the adhesive to fully cure, rendering the adhesive non-tacky. The particles are thereby attached to the primary surface 605 via the cured adhesive. Alternatively, in another embodiment of the invention, the particles may be placed on a shaped thermoplastic that has been heated to a molten state. Upon cooling of the thermoplastic, the particles will remain affixed to and protrude from the primary surface 605 that is defined by the thermoplastic.

The skin-contactable element 105 may include at least one securing surface 109 for contacting mating surface 133 of apparatus 100 (See Figure 1) so as to temporarily secure the skin-contactable element to the mating surface 133 during operation of the apparatus. The securing surface 109 may circumscribe the skin-contactable surface 106 and extend away from the skin-contactable surface 106 to form a rim around the skin-contactable element 105. The securing surface 109 may thereby define, for example, a hollow (not shown) underneath the skin-contactable surface 106 useful for snapping into or against or otherwise mating with mating surface 133 (examples of mating surfaces 133 are shown in Figures 3A-B).

While Figure 6A depicts the raised, non-cutting regions 603 as fine particles, the raised, non-cutting regions 603 may be of varying shapes and dimensions. Other embodiments of the inventive skin-contactable element 105 that include a raised surface useful for enhancing friction and/or managing the movement of fluid at the interface 150 as shown in Figures 6B-6E. The skin-contactable element 105 may include raised regions 631 that protrude from the primary surface 605 and are arranged in a pattern. Examples of suitable patterns include isolated raised rectangular regions 631 as shown in Figures 6B-6C, interconnected raised rectangular regions 631 that define, for example, a plurality of hexagonal recesses as shown in Figure 6D, raised circular regions 631 as shown in Figure 6E, among other patterns. A height differential 611 as shown in Figure 6E, between the primary surface 605 and an adjacent raised region 631 may be sufficiently large to facilitate friction at the interface 150 as well as to facilitate the movement of fluids or other compositions at the interface 150. For example, the height differential 611 may be from about 0.25 mm to about 1 cm.

Figure 6A shows an additional feature that may included in the skin-contactable element 105, a port 606 that is useful for providing fluid compositions (*e.g*., which are, or which include, one or more agents) from within the apparatus 100, such as via delivery system 180 through the port 606 to the skin-contactable surface 106. While one central port 606 is shown, a plurality of ports 606 having various spacing patterns is contemplated.

Various materials may be selected for the skin-contactable element 105. The material forming the primary surface may include, for example, a polymeric material such as an elastomer, such as a thermoplastic elastomer (*e.g*. polyurethane, polyolefin, polyamide, or combinations thereof) or thermoset resin (*e.g*. polyester, polyurethane). The primary surface 605 may be a firm surface (*e.g*., having limited compressibility, such as having a shore hardness of greater than about 20 and/or substantially free of entrapped air such as is present in foam materials), thus facilitating the transfer of mechanical energy from the apparatus 100 to the expanse of skin 102.

The raised regions 631 may have a composition that is substantially identical (*e.g*. both the raised regions 631 and the primary surface 605 are thermoplastic elastomer-based) or, alternatively, a composition that is substantially different than the composition of the primary surface 605 (*e.g*. raised regions of glass particles on a thermoset resin-based primary surface).

The various patterns formed on the skin-contactable surface 106 described above with reference to Figures 6A-6E may be formed by various methods such as casting, extrusion, injection molding, preferential etching, stamping, embossing, and combinations thereof.

In order to enhance the power density that may be delivered by the apparatus 100 through the skin-contactable surface 106, the primary surface 605 may have a projected area (*i.e*., the area of the primary surface as projected onto a plane) that is less than about 20 square centimeters, such as less than about 15 cm², such as less than about 10 cm².

### APPARATUS-ENHANCING AGENTS

Various compositions, hereafter referred to as "apparatus-enhancing agents" may be used to perform some function that manifests when used in conjunction with the apparatus 100. As such, the apparatus-enhancing agents enhance the benefits provided by the apparatus 100 (See Figure 100). One example of an apparatus-enhancing agent is a coupling composition. In one embodiment of the invention, the friction-enhancement is provided to the interface 150 by a providing coupling composition to the interface 150. Sufficient friction enhancement may be provided to (1) enhance the amount of energy that is transferred to the skin-contactable surface 106 and/or (2) to enhance the amount of abrasion delivered to the expanse of skin 102. The coupling composition generally includes a liquid phase, a gel phase, or a semi-solid phase, or combinations of these phases. The coupling composition may be viscous or viscoelastic substance that enhances the coupling of energy from the skin-contactable surface 106 to the skin 102.

The coupling composition may be sebum inactivating. For example, the composition may include an ingredient such as a sebum solvent, such as a non-polar solvent (*i.e*., less polar than water) that removes or renders sebum and other lubricious substances secreted by or present on the skin 102 less slippery. The solvent may be volatile (*i.e*., preferably more volatile than water) and may have a vapor pressure at ambient temperature less than about 25 torr. The solvent may have a boiling temperature at standard pressure of less than about 80 degrees Celsius. The solvent may be water miscible to promote drying of the skin. For example, the solvent may be an aliphatic alcohol such as ethanol, isopropanol, among other solvents useful for removing sebum and oily residue that may be present on the expanse of skin 102. The solvent may be present in the coupling composition in a concentration greater than about 10%, or significantly higher, such as greater than about 25%. Higher concentrations of volatile solvents may be particularly useful to facilitate fast evaporation.

The coupling composition may be capable of stiffening the stratum corneum by removing sebum (such as by including solvents such as those specified above) or by other mechanisms. By stiffening the stratum corneum, the ability of the apparatus 100 to transfer mechanical energy therethrough is enhanced.

The coupling composition may include a constituent that is capable of removing or loosening the stratum corneum, such as, for example, a keratolytic agent such as a hydroxyacid such as an alpha-hydroxy acid or a beta-hydroxy acid. Suitable hydroxyacids include lactic acid, citric acid, glycolic acid and salicylic acid, among others. By removing or loosening the stratum corneum mechanical energy can more readily be delivered to layers of the expanse of skin 102 underneath.

In one embodiment, the coupling composition is a particulate in a dry state. For example, the coupling composition may be free of physically-bound water or have a concentration of water that is less than about 1% such as less than about 0.2%. One example of a suitable dry particulate composition is ascorbic acid. Other suitable dry particulate compositions include porous hard particles such as porous acrylic particles (having a particle size of, for example, about 10 microns to about 200 microns) that have one or more benefit agents (suitable benefit agents are described in the section entitled "Benefit Agents" below) absorbed onto or embedded therein. One example of a suitable porous hard particle that may be used to deliver benefit agents to the skin is available from Advanced Polymer Systems of Redwood City, California under the trade name MICROSPONGE™.

The coupling compositions may be substantially or completely free of surfactants (such as those defined as such in McCutecheon's Emulsifiers and Detergents, North American Edition) or other non-volatile ingredients, such as may leave behind a slippery residue that detracts from the ability to couple mechanical energy into the expanse of skin 102. The coupling composition may include other functional ingredients that do not detract from its ability to couple energy. For example, the coupling composition may include fillers, minerals, certain polymers, chelating agents, fragrances, dyes, and the like. The coupling composition may be provided to the interface 150 by applying the composition to the expanse of skin 102 by hand, using the chemical delivery sub-assembly 180, or by means described below in the section entitled, "Delivery of Agents To The Skin."

The coupling composition may be free of tacky substances, *i.e*., free of adhesive substances or substances that, alone or in combination, function to provide tack. Examples of tacky substances that may be excluded from the coupling composition include monomeric, oligomeric or polymeric compounds having a molecular weight distribution suitable for providing a releasably tacky surface such as when combined with tackifying resins. By excluding tacky substances from the coupling composition, it may be possible to facilitate the delivery of various benefit agents to the expanse of skin 102 without causing the benefit agents to become entrapped in the tacky substance.

Examples of such monomeric, oligomeric or polymeric compounds that may be excluded are include acrylics (including monomers such as methacrylic acid, acrylic acid, as well one or more of as various ester functionalies) in a solvent, emulsion or radiation-cured syrup form; natural or synthetic rubbers such as polyisoprene or such as KRATON synthetic rubber-based adhesives having thermoplastic elastomeric components from Shell Chemical Company (Houston, TX); polydimethylsiloxanes, polyurethane elastomers, or other suitable skin-contact adhesives. The monomeric, oligomeric or polymeric compounds that may be excluded include those compounds above that are incompletely cured.

Other compounds that may excluded are tackifying resins such as natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof.

The coupling composition may be applied to the expanse of skin 102 prior to contacting the expanse of skin 102 with the skin-contactable surface 106 (*i*.*e.,* the coupling composition may be a "pre-treatment composition"). Alternatively or in addition, the coupling composition may also be applied during the contacting the expanse of skin 102 with the skin-contactable surface 106.

While the coupling composition described above serves the function of enhancing friction at the interface 150, the coupling composition may alternatively or in addition serve the function of rendering the expanse of skin 102 that is being treated with the apparatus 100 substantially homogeneous with respect to its surface properties (*e.g*. the coupling composition is also a "homogenizing composition"). As such, when the expanse of skin 102 is being contacted with the skin-contactable surface 106, the entire expanse of skin 102 responds similarly in terms of its ability to couple energy to the sub-layers of the skin below the stratum corenum.

In another embodiment of the invention, the homogenizing composition does not enhance the coupling of energy to the expanse of skin 102 and only has a homogenizing function absent a coupling function. This homogenizing composition may be used to create a surface on the expanse of skin 102 that is somewhat independent of the particular individual, thereby making treatment of the skin via the apparatus 100 more predictable from user to user. The homogenizing composition may be, for example, a lubricious composition such as one comprising glycerin, various moisturizers or emollients, and the like, and may be delivered in a manner and timing similar to the coupling composition described above.

### POWER OUTPUT TO THE SKIN

The skin-contactable surface 106 may be adapted to provide a power output to the skin that is sufficient to provide one or more skin benefits. In one embodiment of the invention, the skin-contactable surface imparts a power output to the skin that is greater than 0.2 watts, such as greater than about 0.25 watts, such as greater than about 0.4 watts. Such power outputs may be particularly beneficial in providing a faster onset or greater magnitude of benefits (e.g., rejuvenation) to skin through mechanical transfer of energy to the skin as well as by enhanced performance of a benefit agent that is in contact with the skin when said power output is applied. In one embodiment of the invention, the skin-contactable surface imparts an output power density to the skin (output power to the skin divided by area of the skin-contactable surface 106) that is greater than 110 watt/square meter (W/m²) such as greater than about 125 W/m² such as greater than about 200 W/m². The apparatus 100 may be of light weight to allow the user to position the apparatus 100 against the expanse of skin 102, to move the apparatus 100 across the expanse of skin 102, and to hold the apparatus 100 for a period of time that is sufficient to treat the entire expanse of skin 102. In one embodiment of the invention, the time period is between greater than about 1 minute, such as between about 1 and about 5 minutes for an expanse of skin having an area of about 25 square centimeters.

### DETERMINATION OF POWER OUTPUT

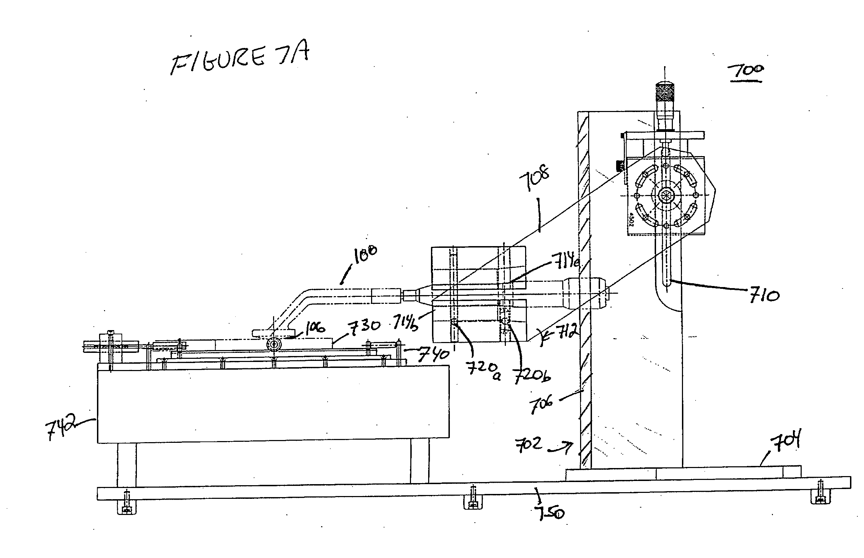

Power output to the skin and power density output of the apparatus may be determined using the following method. A side view of an exemplary test apparatus 700 is depicted in Figure 7A. The test apparatus 700 includes a stand 702 for supporting an apparatus to be tested such as the apparatus 100. The stand 702 generally includes a base 704 for resting on a support 750 and a L-shaped, vertical rise 706 perpendicular to the base 702, from which an arm 708 extends. The arm 708 is, for example, rotatably attached to the rise 706 such that an angle 712 may be adjusted. Furthermore, the arm 708 is coupled in a slideable manner to the rise 706 so that the height of the arm 708 may be adjusted. For example, the arm 708 may be attached to the rise 706 via screws that are placed through a slot 710 in the rise 706.

The apparatus 100 is placed in between a pair of grips 714a, 714b that are about 3 1/4 inches long for firmly holding and stabilizing the apparatus 100. The grips 714a, 714b generally have a surface that is capable of holding the apparatus 100 tightly and without the apparatus 100 sliding or slipping during the test. The grips 100 preferably have an elastomeric surface to facilitate gripping of the apparatus 100, and simulating a human hand performing this function. The apparatus 100 is held within the grips 714a, 714b by providing a clamping force such as is achieved by tightening two holding screws 720a, 720b (located within slotted holes) having a screw diameter of 0.375 inches and 16 threads per inch to a torque of 2 ounce-inches.

A balance 740 having a balance pan is placed upon and rigidly affixed to a horizontal surface, such as that of a secure stand 742, below the grips 714a, 714b. A test skin 730 (described below) of substantially circular shape and about 5.5 inche diameter when viewed from the top is placed on the balance pan and secured using a strong double-sided tape or other suitable securing device that is strong enough to immobilize the bottom surface of the test skin 730 throughout the test.

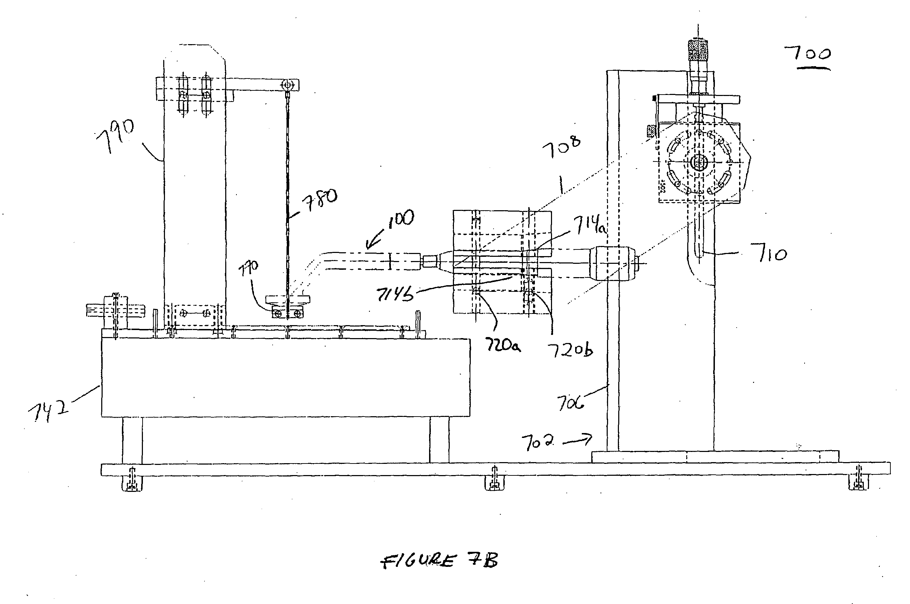

The apparatus 100 is electrically coupled to a voltmeter and ammeter (meters are not shown in Figure 7) in order to monitor the current that is being drawn by the motor 130 during the duration of the test.

By adjusting the height of the arm and the angle 712, the skin contactable element 106 of the apparatus 100 to be tested is brought into direct contact with an outer surface of the test skin 730. A point that is in the approximate center of the skin contactable surface 106 is positioned over the approximate center of the test skin 730 (e.g., no external compositions, such as creams, are placed between the skin contactable surface 106 and test skin 730). If the skin contactable surface 106 is substantially flat, then the skin contactable surface 106 should be substantially positioned as flat against the test skin 730 as possible. The skin contactable surface 106 is lowered to a point at which the balance registers a normal force that simulates a typical force provided by a user holding the apparatus 100, such as, for example, 170 grams.

In order to determine the output power to the skin by the test apparatus 700 (*i.e.,* the power that is not transmittable into the test skin 730), two separate tests are required. Firstly, as shown in Figure 7B, the test apparatus 700 is modified to record a baseline current and baseline voltage. The skin-contactable surface 106 is rested against a plate 770 that is suspended from an inelastic cable 780 (*e.g*. steel or generally non-stretchable fiber) that is coupled to the balance 740 via an arm 790 that sits on the balance 740. The apparatus 100 is otherwise positioned as described above and in a manner so as to register 170 grams on the balance. Power is provided to the apparatus 100, and the baseline current and baseline voltage are recorded. Baseline power is calculated as the product of baseline voltage times baseline current. Thereafter, in a second test, the skin contactable surface 106 is brought into contact with the test skin 730 such that the desired mass is recorded on the balance and a test current and test voltage are recorded. The product is calculated in order to determine the test power. In general, the test power will be greater than the baseline power, since the current drawn by the apparatus increases as the apparatus consumes more power (*e.g*. more current) in order to actuate the skin-contactable surface 106 under the load due to the test skin 730. Baseline power is subtracted from test power in order to determine the power output to the skin. By subtracting out the baseline power, the power that is consumed by the test apparatus and not provided to the skin is removed from consideration when comparing apparatuses. The above process steps, including the determination of baseline power, is repeated for all of the apparatuses to be tested.

The synthetic skin is constructed using the following method. A bottom layer is formed using AQUATRIX II, a hydrophilic polymer gel, available from Hydromer Inc. of Somerville, NJ. Part A of AQUATRIX II, a polyvinylpyrrolidone-based solution is mixed in equal parts with Part B of AQUATRIX II, a chitosan-based solution at room temperature. The mixture is stirred at ambient temperature and a vacuum is applied to the headspace of the mixture to minimize the entrapment of air bubbles. The mixture is mixed for about fifteen minutes using a stirring shaft, at slow speed until it starts climbing up the shaft of the stirrer. The resulting gel is then poured into a 5 1/2" diameter petri dish to form a bottom skin layer to a thickness of about 0.5 inches.

A layer of NUGEL A (crosslinked polyvinyl pyrrolidone), commercially available from Johnson & Johnson Medical Inc., of New Brunswick, NJ is then removed from its packaging and both outer layers of plastic are discarded. The NuGel A is cut to a 5 ½" diameter and placed onto the bottom skin layer to form a middle skin layer.

A layer of VITRO-SKIN, commercially available from IMS Testing Group of Milford, Connecticut is cut to a 5 ½" diameter piece and then placed on the middle skin layer to form an upper layer of a tri-layer synthetic skin laminate. The laminate is then irradiated with Cobalt 60 radiation. The dose of radiation is targeted to be 25 kiloGray (2.5 megarads), which may be delivered in a time period of about 150 minutes. A suitable machine that may be used to irradiate the laminate is the Gamma Cell 220, commercially available from Nordion International Inc., of Kanata, Ontario, Canada.

### DELIVERY OF AGENTS TO THE SKEIN

Various compositions such as the apparatus-enhancing agents (*e*.*g.* coupling composition, the homogenizing composition), and the benefit agents may be delivered to the expanse of skin 102 via on or more application mechanisms. These application mechanisms include conventional rubbing, pumping and/or spraying a thin liquid, gel or cream onto the expanse of skin 102. Other suitable methods delivery include pumping or injecting the composition via the chemical delivery sub-assembly 180, such as to through port 606 as shown in Figure 6A.

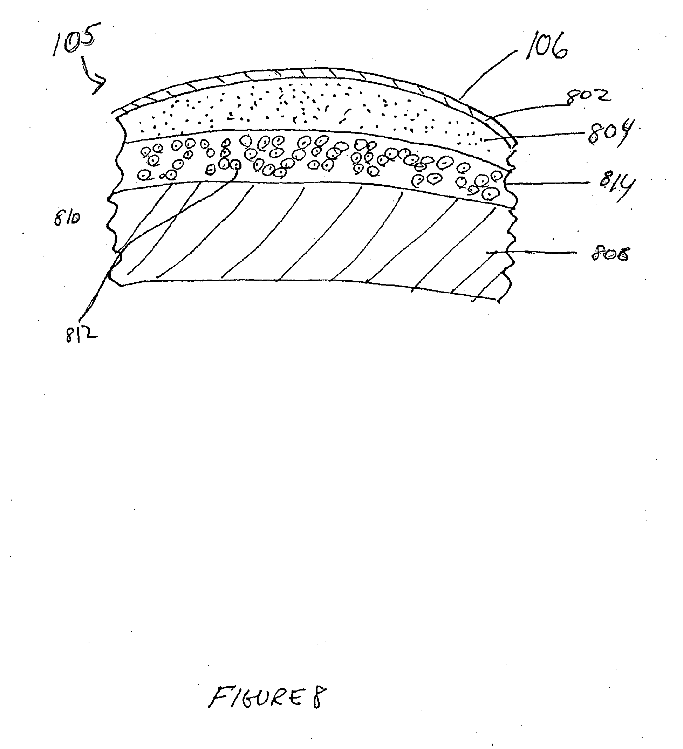

Referring to Figure 8, in one embodiment of the invention, the skin-contactable element 105 includes a skin-contactable surface 106 that is substantially free of tack. In one embodiment, the skin-contactable surface 106 does not include adhesive substances or substances that, alone or in combination, function to provide tack. Suitable tacky materials that may be excluded from the skin-contactable surface 106 include those discussed previously (tacky polymer materials and tackifying resins), with reference to coupling compositions discussed in the section entitled, "APPARATUS-ENHANCING AGENTS." For example, if the skin-contactable surface is tested using the Probe Tack Tester (commercially available from Testing Machines Inc. of Amityville, NY), using TMI Model 80-02-01 fitted with the "A" annular weight and "F" auxiliary weight accessories, set to a speed of 1 centimeter per second, and tested using ASTM D 2979, the resulting tack reading will be less than about 20 grams such as less than about 5 grams).

The skin-contactable element 105 may include an agent that is configured for release from the skin contactable element in order to contact or upon contacting the expanse of skin. The agent is generally capable of being transferred from the skin-contactable element 105 to the expanse of skin 102 when the skin-contactable surface 106 is placed in contact with the expanse of skin 102. The agent may be positioned within the skin-contactable element 106 so as to contact the expanse of skin 102 after a pre-determined range of time that the skin-contactable element has been used. Note that by excluding tacky materials from the skin-contactable surface 106, the delivery of agents to the expanse of skin 102 is facilitated. This is because various agents may tend to become entrapped within the tacky substance, reducing delivery to the expanse of skin 102.

In one embodiment of the invention, the skin-contactable element 105 includes at least a portion of the agent that is configured to be expressed through a porous material (such as the porous sheet 108 shown in Figure 3B) in a manner that is sensitive to a pressure that is applied between the expanse of skin 102 and the skin-contactable surface 106.

One suitable skin contactable element 105 is multi-layered, as depicted in Figure 8. In this embodiment of the invention, the agent is spaced apart from the skin-contactable surface 106 by a control layer 802. The control layer 802 may serve to, for example, protect an agent 804 from premature degradation (*e.g*. oxidation). Separately, or in addition to protection, the control layer 802 may serve to deliver the agent 804 at a controlled or steady rate, or to deliver the agent 804 after a range of time that the skin-contactable element has been used. The control layer 802 may include a material that facilitates gradual release of the agent 804 over time under pressure, at a particular temperature, or when subject to a particular chemical environment. For example, the control layer 802 may include a rate controlling membrane such as a porous or nonporous polymeric material such as an ethylene vinyl acetate-based material. The control layer 802 may include the agent to be delivered or the agent may be positioned entirely underneath the control layer 802 such that it may, for example, diffuse through the control layer 802 during operation of the device.

In one embodiment of the invention, the control layer 802 is a layer that dissolves at a controlled rate. For example, the control layer may be a layer of metal such as silver, zinc, copper, or combinations thereof that slowly ionizes or dissolves (such as when subject to an acidic medium), thereby providing a steady or controlled dose of metal ions to the skin. This embodiment may be particularly usedul for treating acne. The layer of metal may be, for coated such as by electroplating, electroless plating, thermal evaporation, and the like onto an underlying surface.

At least a portion of agent 804 is may be positioned beneath the skin-contactable surface 106, the portion configured to contact the expanse of skin 102 after a pre-determined delay period between a time at which the skin contactable surface contacts the expanse of skin and a time at which the agent contacts the expanse of skin.

The control layer 802 may be configured to denature such as by hydrolysis over the delay period, wherein after the delay period, contact of the agent with the expanse of skin 102 is facilitated. For example, the control layer 802 may denature by becoming more porous or by disintegrating. Controlled disintegration of the control layer 802 may be achieved by, for example, by constructing the control layer 802 such that it is mechanically wearable during operation of the apparatus 100. In this embodiment of the invention, the initial skin-contactable surface 106 is worn away progressively to reveal a fresh skin-contactable surface that advances toward the interior of the skin contactable element 105 as the control layer 802 is worn away. In this embodiment of the invention, the control layer 802 may be a wearable layer of, for example, a plastic, an elastomeric, or a fibrous or other porous material. In these embodiments the control layer may be a wearable fatty material such as a triglyceride, a partial glycerides, a fatty alcohol, a fatty ester and the like or a wearable polymer such as polyethylene glycol, polyvinyl alcohol, and the like that may, for example printed onto a fibrous base. The wearble polymer have abrasive particles or dyes positioned within.

In an alternative embodiment of the invention, the control layer 802 may be removable prior to operation, such as by being peeled off or otherwise detached by hand, such as from a perforated interface (not shown). Other suitable configurations of the skin-contactable element 105 have an agent that is formed, incased or included within or otherwise at least partially affixed to the skin-contacting element 105. For example, the agent may be positioned within a void or pocket (not shown) that is located within the skin-contacting element 105, it may be compounded into the materials that form the skin contacting element 105. The agent may be encapsulated by, for example, a polymer, a liposome, among other encapsulants so as to increase its shelf life within the skin-contactable element 105. In another embodiment of the invention, the agent is positioned within the raised regions or so as to be delivered from only select portions of the skin-contactable surface 106.

Referring again to Figure 8, the agent may be confined toan active layer 804 of the skin-contactable element 105 that is, for example, positioned beneath the control layer 802. While Figure 8 depicts skin-contactable element 105 having one benefit layer, multiple benefit layers may be included, each having any number of benefit agents that are compatible with one another.

The agent may be formulated with other benefit agents and/or with other ingredients to form a composition that may be a solid (*e.g*., a particulate, a free-standing film, a plastic or fibrous medium such as a non-woven material, and the like), a liquid (*e*.*g*,. water-thin or viscous, including pastes, creams, emulsions), or combinations thereof. The active layer 804 may be positioned upon other layers such as an indicator layer 814 (described below) or a structural layer 808 (a layer that does not include any agents to be delivered to the interface 150).

The skin-contactable element 105, if multi-layered, may be formed using various plastics or elastomer processing techniques as discussed above, including extrusion, injection molding, casting, and the like, as well as lamination or mechanical means to join the multi-layers together to form the skin-contactable element 105. The various layers of the skin-contactable element 105 may have thicknesses that are variable, such as from about 0.1 microns to about 5 mm.

### SIGNALING MARKER

Referring again to Figure 8, in one embodiment of the invention, the skin-contactable surface 106 includes at least one signaling marker 810 (such as may be included in a signaling layer 814) that is at least partially affixed to the skin-contactable element. The signaling marker 810 is adapted to provide a change in a sensation that is discernable to a user proximate the apparatus after a period of time of operation of the apparatus, such as when the signaling marker 810 is exposed. The sensation may be tactile, olfactory, thermal, visual, auditory, or combinations thereof. The at least one signaling marker 810 is capable of being delivered to the skin when the skin-contactable surface is applied the expanse of skin 102.

The signaling marker 810 may be exposed by a gradual wearing down of the skin-contactable element 105 during use of the apparatus 100. The signaling marker 810 may be otherwise be delivered to the expanse of skin 102 as described above for the agent in the above section "DELIVERY OF AGENTS TO THE SKIN" (*e.g*. within or under a separate control layer).

The signaling marker 810 provides a visual, auditory, tactile or olfactory cue to the user that it is time to initiate a change in operation of the apparatus 100. For example, the signaling marker 810 may provide a tactile sensation to the subject that is different than a previous sensation provided by the skin-contactable element 105 before the signaling marker 810 was exposed.

While Figure 8 depicts the signaling marker 810 as positioned in a signaling layer 814 that is below the agent 804, this need not be the case. For example, in one embodiment of the invention, the skin contactable surface 106 may have regions such as stripes, dots or shaped patterns or indicia of abrasive particles positioned within or protruding from a matrix. The matrix may include a polymer (e.g., polyethylene glycol and the like) or a fatty material (e.g., a fatty ester, alcohol, and the like). The abrasive particles may be of varying compositions (e.g., silica, carborundum, smooth glass, polyethylene, etc.). The abrasive particles and matrix may be coated or printed upon a fibrous or thermoapertured film substrate that may be removable or replaceable from the skin-contactable element. In use, upon contact with the skin for a period of time, the matrix may wear away, causing the abrasive particles to detach. After a period of time, a substantial portion of the abrasive particles may detach, signaling to the user that it is time to replace the worn substrate with a fresh substrate (having fresh matrix and fresh abrasive particles thereon). Note that in this embodiment of the invention in which abrasive particles serve as the signaling marker 810, the wearable matrix may include benefit agent as well as abrasive particles.

In another embodiment of the invention, the skin contactable element 105 may include a replaceable web of fibrous material that is designed, upon excessive use, to tear or otherwise mechanically degrade in a way that the user can feel a change in tactile or see a visual change in sensation, thereby recognizing that it is time to change the change the fibrous web to a fresh one.

The sensation imparted by the signaling marker 810 may indicate that it is time to replace the skin-contactable element 105 with a fresh one in order to maintain efficacy of treatment or to provide a fresh, hygienic surface 106. In another embodiment of the invention, the signaling marker 810 may indicate that it is time to adjust motion (*e.g*., the amplitude or frequency of the skin-contactable surface 106) of the device, time to change, add, or adjust the type or flow rate of the agent, and the like.

In one embodiment of the invention, the signaling marker 810 is encapsulated within an outer wearable coating 812. The coating 812 may include any material, such as an acrylate polymer, that is capable of gradually wearing away throughout the operation of the apparatus 100. The signaling marker 810 may include any material that has a visual, tactile, thermal, or olfactory properties that are discernable when the coating is compromised and the signaling marker 810 is released. Suitable examples of materials that may be included in the end-point marker include fragrances and other volatile, odorous compounds; cosmetically acceptable dyes or pigments; cooling compounds; warming compounds such as (anhydrous) inorganic salts that release heat when contacted with moisture, and the like.

The signaling marker 810 may provide its signaling function as a result of interaction with the expanse of skin 102 and/or with the mechanical energy imparted thereto by the skin contactable surface 106. The signaling marker 810 may have one or more properties that are sensitive to the motion or pressure provided by the skin-contactable surface 106, particularly when the signaling marker 810 is exposed. For example, the signaling marker 810 may experience a change in viscosity (such as a shear-thickening or shear-thinning material may experience), thereby generating a tactile sensation; an odor, or absorb or release thermal energy when subject to vibration, shear, or pressure, in order to communicate to the subject that the treatment or a particular stage thereof is complete. The signaling marker 810 may be located within a separate layer such as layer 812 depicted in Figure 8. The layer 812 may be beneath the active layer 804.

### CHEMICAL DELIVERY SUB-ASSEMBLY

Referring again to Figure 1, the user output assembly 104 may include chemical delivery sub-assembly 180 for delivering one or more agents to the skin, for example, via the skin-contactable surface 106. Referring again to Figure 1, the chemical delivery sub-assembly 180 may be designed to deliver one or more fluid compositions, each including one or more agents, apparatus-enhancing agents or other compositions to the interface 150. In order to perform this function, the chemical delivery sub-assembly 180 may include various components such as detachable cartridges or other storage containers 185 for storing various fluids, valve 181 or membranes (not shown) for controlling the flow rate of the fluids to the interface 150, pumps (not shown) for facilitating the delivery of the fluids to the interface 150, conduits 183 for transporting the fluids to the interface 150 as well as heaters for rendering the fluids flowable or active (not shown). Valves 181 may be electromechanically coupled to controller 240 such that the flow of agents can be pre-programmed or altered based upon information from the sensing element 270.

### INDICATOR

While the signaling marker 810 is described above as associated with the skin-contact surface 106, other indicators that are not so associated may be employed to communicate with the user of the apparatus 100. Referring to Figure 1, the user-output assembly 104 may optionally include indicator 245, generally spaced apart from the skin contactable surface 106, that permits the apparatus 100 to communicate with the user. The indicator 245 may be, for example, a visual light or display such as a LED or LCD, an auditory display such as may provide a "beep" or other indicating sound, or a vibrating or heating element that may be positioned within the handle 122 in order to communicate to the user. The indicator 245 emits a stimulus that is readily detectable by the user. The indicator 245 may, for example, be used to alert the user that the user is applying the skin-contactable surface to the skin with a pressure on the skin-contactable surface 106 that is too great. Similarly, the indicator 245 may be used to communicate to the user that the motor 130 is vibrating too fast, that the temperature on the skin is too high, that the skin-contactable element 105 is worn and requires replacement, among other states of the apparatus 100 or the skin 102 that would be desirable for the user to be aware of.

### CONTROLLER

Referring to Figure 1, to facilitate control of the apparatus 100, microprocessor controller 240 may be electrically coupled to the various components of the user output assembly 104 among other components of the apparatus 100. The controller 240 includes, for example, a central processing unit (CPU) 275, a memory 280, and support circuits 285 for the CPU 275. The CPU 275 may be one of any form of a general purpose computer processor that may be used for controlling various pieces of industrial or consumer-product electromechanical process equipment. The memory 280 is coupled to the CPU 275. The memory 280 or a computer readable medium may be one or more of readily available memory such as random access memory (RAM), read only memory (ROM), hard disk, floppy disk, or any other form of digital storage. The support circuits 285 are coupled to the CPU 275 for supporting the CPU 275 in a conventional manner. These circuits may include cache, power supplies, clock circuits, input/output circuitry, and the like. A skin treatment process may be stored in the memory 280 as a software routine. A software routine may also be stored and/or executed by a second CPU (not shown) that is remotely located from the hardware controlled by the CPU 275.

The software routine, when executed may transform the general purpose computer into a specific purpose computer (controller 240) that controls the operation of the motor 130 such that the skin treatment process is performed. The controller 240 is coupled to the sensing element 270 and/or the receiving element 255 and receives data therefrom. Based upon this data, the controller 240 provides action instructions to the user output assembly 104 (*e.g*., the mechanical energy delivery sub-assembly 112, the chemical delivery sub-assembly 180, the indicator 245, or combinations thereof).

The controller 240 is capable of controlling one or more aspects of the apparatus 100 including, for example, the amplitude and frequency of motion of the skin contactable surface 106 in order to generate a particular waveform of motion. By "waveform," it is meant any particular time-varying distribution or pattern of amplitude and frequency of motion (*e.g*., reciprocating, orbital, and the like) of the skin-contactable surface 106.

The waveform may be generated by one or more time-varying currents and/or voltages provided to the motor 130, which are then communicated to the transfer member 125. In one embodiment of the invention, the motor 130 is a linear motor that is coupled to at least one reversing circuit that is used to control or adjust the waveform. The controller 240 may also be capable of controlling the selection of agents to be delivered to the interface 150 and the rate of delivery of such agents, such as by being electrically coupled to valves 181 of the chemical delivery system 180. The controller may also be capable of controlling a state (*e*.*g.* on/off) of the indicator 245, such as by being electrically coupled thereto.

The controlling software of controller 240 may be programmed via the receiving element 255 by coupling to a standard port 250 (see Figure 1) so as to customize it for specific users, as described below.

### RECEIVING ELEMENT AND SENSING ELEMENT

In one embodiment of the invention as shown in Figure 1, the apparatus 100 includes the receiving element 255 coupled to the controller 240. The receiving element 255 is capable of receiving data such as user-attribute data, including, for example, age, sex, height, weight, body mass index and other personal characteristics that may serve correlate to one or more skin properties of the subject. The receiving element 255 may be an external interface that provides a means of connecting the controller 240 to an external data source (not shown in Figure 1) for the purpose of programming the controller 240. The receiving element 255 may be a USB port or RS-232 connector, for example. The receiving element 255 may be coupled to the controller 240 via, for example, the standard port 250.

In one embodiment of the invention, the apparatus 100 includes a sensing element 270 in communication with the skin-contactable surface 106. Whereas the receiving element 255 is generally used to provide information to the controller 240 based upon "pre-programmed" user attribute data, the sensing element 270 is used to provide information to the controller 240 during operation of the apparatus 100. The sensing element 270 is capable of sensing a state of at least one or more property associated with the skin 102. The sensing element 270 is, for example, electrically coupled to the controller 240. The controller 240 may provide action instructions to the user output assembly 104, the instructions dependent upon the one or more properties sensed by the sensing element 270.

The sensing element 270 is any of several sensors that provide data from skin-contactable surface 106, or expanse of skin 102, to the controller 240. The sensing element 270 may sense one or more of such properties as a pressure (*e.g*., a pressure between the expanse of skin 102 and the skin-contactable surface 106), a chemical property, an optical property, or a thermal property. The sensing element 270 may be, for example, at least one thermistor, pressure transducer, chemical sensor, photodiode, or combinations thereof. Controller 240 may use sensing element 270 data to further customize current and/or voltage waveforms according to specific users' needs and conditions.

The sensing element 270 and/or the receiving element 255, if present, are in communication with the controller 240 and the user output system 104 and cooperate therewith. There are numerous ways in which this cooperation may take place. For example, the sensing element 270 may sense, for example, a load state (*e.g*. the force exerted upon the skin-contactable surface 106 by the expanse of skin 102 to which it is in contact). Information regarding this state is communicated to the controller 240. The controller 240 may then send a signal to a component of the user output system 104 such as the motor 130 to modify the existing waveform that will be output by the skin-contactable surface 106. Similarly, a signal may be sent to the indicator 245 such that it may emit light, an audible tone, or a vibrational stimulus to indicate to the user that the load has fallen within or outside a pre-determined range, *e.g*. below about 0.1 psi or greater than about 20 psi. The sensing element 270 may alternatively sense a state of surface temperature of the expanse of skin 102 and, as a result, modify the waveform or provide a signal to the indicator 245 as appropriate (*e.g*. the motor 130 may be shut off if the temperature is greater than a critical temperature). In another embodiment of the invention, the sensing element 270 senses an optical property such as skin reflectivity, transmissivity, fluorescence, light scattering, and combinations thereof and modifies the waveform as appropriate.

### WAVEFORM CONTROL

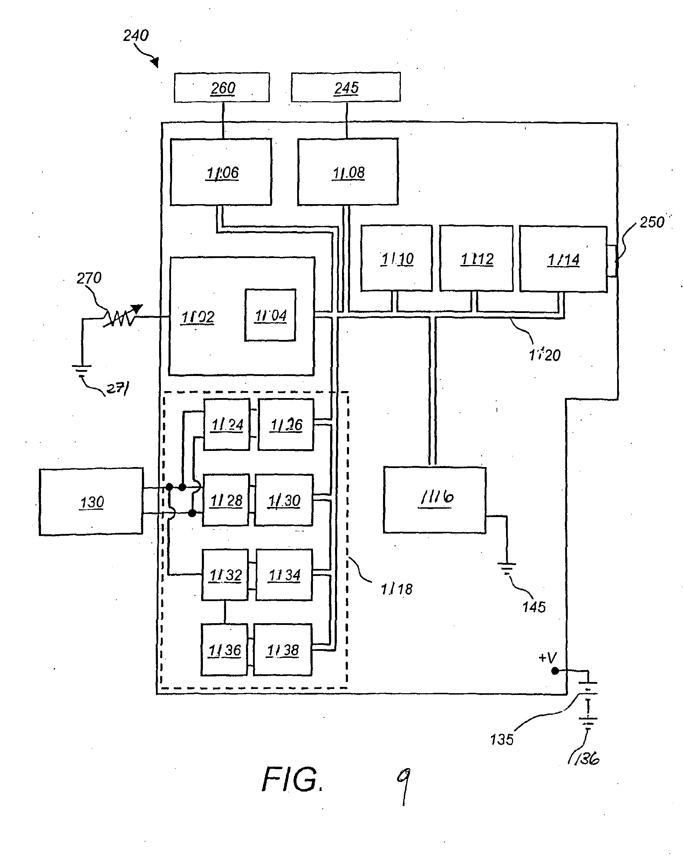

In one embodiment of the invention, a set of waveform instructions that is provided by the controller 240 to the motor 130 is initiated, modulated or adjusted in response to information provided by the receiving element 255, the sensing element 270, or combinations thereof. Figure 9 illustrates one embodiment of the apparatus 100 that is useful for initiating or modifying waveforms.

Figure 9 illustrates a block diagram of controller 240, which may suitably control the operation of the apparatus 100. Figure 9 also shows the controller 240 electrically connected to the motor 130, the energy storage element 135, the indicator 245, the standard port 250, the input 260, and the sensing element 270. The sensing element 270 is grounded using ground 271 and the energy storage element 135 is grounded using ground 136.

In this embodiment of the invention, the controller 240 includes a sensor input 1102, an analog-to-digital converter (A/D) 1104, an input controller 1106, an indicator driver 1108, a random-access memory (RAM) 1110, an erasable programmable read-only memory (EPROM) 1112, an external interface driver 1114, and a microcontroller 1116. Furthermore and associated with the control of motor 130, controller 240 includes a motor controller region 1118 that includes a voltage source (V_{S}) 1124, a V_{S} digital-to-analog converter (D/A) 1126, a voltage meter (V_{M}) 1128, a V_{M} A/D converter 1130, a current meter (I_{M}) 1132, an I_{M} A/D 1134, a current source (I_{S}) 1136, and an Is D/A 1138.

Communication between microcontroller 1116 and A/D 1104, input controller 1106, indicator driver 1108, RAM 1110, EPROM 1112, external interface driver 1114, V_{S} D/A 1126, V_{M} A/D 1130, I_{M} A/D 1134, and Is D/A 1138 is accomplished via an electrical connection to a data/address bus 1120, which may be a standard bi-directional data and address communications bus. Furthermore, all elements of controller 240 receive voltage supply (+V) from storage element 135 via a common power bus (not shown).

Sensor input 1102 serves as the electrical interface between controller 240 and sensing element 270. Sensor input 1102 detects the output of sensing element 270 and subsequently feeds A/D 1104, which performs a well-known analog-to-digital conversion function for converting the analog output of sensor input 1102 to a digital signal suitable for feeding to data/address bus 1120 and for subsequent interrogation by microcontroller 1116.

Input controller 1106 serves as the electrical interface between controller 240 and external input 260. Input controller 1106 detects the state of any buttons or switches associated with input 260 and transfers this digital information to data/address bus 1120 for subsequent interrogation by microcontroller 1116. Indicator driver 1108 serves as the electrical interface between controller 240 and external indicator 245. Indicator driver 1108 provides the drive circuitry for any visual, audible, or tactile stimulation devices (such as indicator 245) associated with indicator 245. Indicator driver 1108 operates under the control of microcontroller 1116 for driving external indicator 245.

RAM 1110 may be any standard RAM device for storing data. A typical capacity of RAM 1110 may range, for example, between 8 K and 64 K. RAM 1110 is used for real-time data inputs and outputs for the software program (not shown) executed by microcontroller 1116 using software code stored in EPROM 1112.

EPROM 1112 is any standard erasable PROM device, with the ability to be programmed at any point from original manufacture or upon purchase of apparatus 100 by a user. EPROM 1112 is used for storing control software code for use by microcontroller 1116.

External interface driver 1114 serves as the electrical interface between controller 240 and standard port 250. External interface driver 1114 is any of a set of drivers that allow an interface between an external programming device and microcontroller 1116 using, for example, a USB or RS-232 interface.

Microcontroller 1116 is any conventional microprocessor device, such as an 8-bit microcontroller, that provides software and hardware control for managing all elements and operations of controller 240.

Collectively, V_{S} 1124, V_{S} D/A 1126, V_{M} 1128, V_{M} A/D 1130, I_{M} 1132, I_{M} A/D 1134, Is 1136, and Is D/A 1138 provide hardware control for all functions of motor 130. These elements provide a voltage source, voltage measurement, a current source, and current measurement for motor 130.

More specifically, V_{S} 1124 provides a voltage source to motor 130. V_{S} D/A 1126 provides digital-to-analog data conversion for V_{S} 1124, whereby the digital data received from data/address bus 1120 is suitably converted by V_{S} D/A 1126 to an analog output for use by V_{S} 1124. The voltage output value of V_{S} 1124 is thus set under the control of microcontroller 1116.

V_{M} 1128 provides a voltage measurement function for motor 130. V_{M} A/D 1130 provides analog-to-digital data conversion for V_{M} 1128, whereby the analog signal received from V_{M} 1128 is suitably converted by V_{M} A/D 1130 to digital data for connecting to data/address bus 1120 and for subsequent interrogation by microcontroller 1116.

I_{M} 1132 provides a current measurement function for motor 130. I_{M} A/D 1134 provides analog-to-digital data conversion for I_{M} 1132, whereby the analog signal received from I_{M} 1132 is suitably converted by I_{M} A/D 1134 to digital data for connecting to data/address bus 1120 and for subsequent interrogation by microcontroller 1116.

Is 1136 provides a current source to motor 130. Is D/A 1138 provides digital-to-analog data conversion for Is 1136, whereby the digital data received from data/address bus 1120 is suitably converted by Is D/A 1138 to an analog output for use by I_{S} 1136. The current output value of Is 1136 is thus set under the control of microcontroller 1116.

All elements of controller 240 may be embedded into a single microelectronics chip or may be separate microelectronics chips that are connected by a common bus. Each element of controller 240 has an address that is understood by microcontroller 1116. Each element has the ability to send and receive data via data/address bus 1120. For example, sensor input 1102 receives analog data from sensing element 270, converts the data to digital form, and sends the digital data to microcontroller 1116, where it is processed. After processing data from sensor input 1102, microcontroller 1116 may slow the rotational speed of motor 130 by sending a command to V_{S} 1124 to reduce the voltage supplied to motor 130.

In one example mode of operation, control software code data for use by microcontroller 1116 is loaded into EPROM 1112 via standard port 250 and external interface driver 1114. Microcontroller 1116 receives software control instructions from EPROM 1112. For example, EPROM 1112 may instruct microcontroller 1116 to move a single piece of data from EPROM 1112 for output to indicator 245. Microcontroller 1116 executes the data transfer function, sends the data to indicator driver 1108, and instructs indicator driver 1108 to activate any visual, audible, or tactile stimulation devices of indicator 245.

In one example of motor control operation, V_{M} 1128 measures the voltage across motor 130 and I_{M} 1132 measures the current through motor 130. Microcontroller 1116 may interrogate these measurements and use this data to adjust the waveform data provided to motor 130. For example, if the user of apparatus 100 applies an excessive load on skin-contactable element 105, the current provided to motor 130 from I_{S} 1136 is increased accordingly under the control of microcontroller 1116. I_{M} 1132 measures the current increase and sends the current data to microcontroller 1116, which may then alter the waveform profile accordingly.

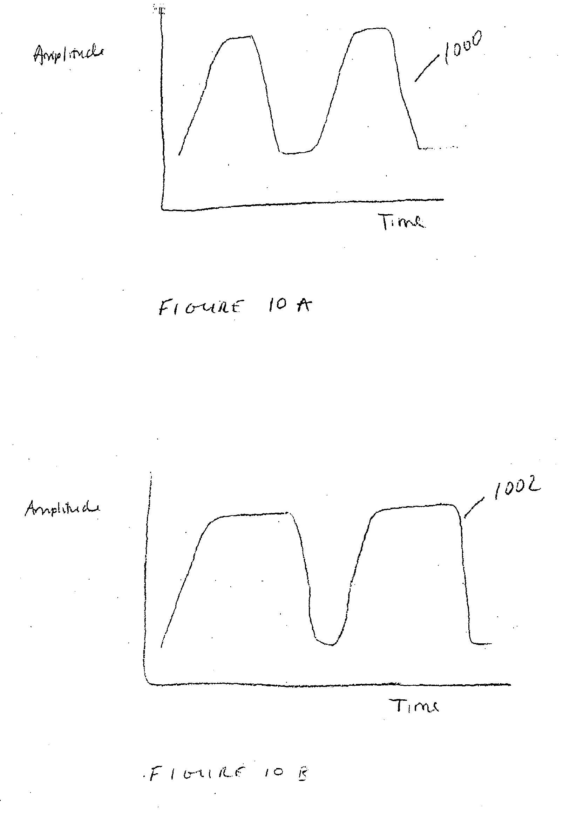

For example, Figures 10A-10D show a series of exemplary waveforms 1000, 1002, 1004 and 1006 respectively, that may be produced by varying the levels of voltage and/or current levels provided to motor 130 from energy storage element 135 over a period of time. The vertical axis of each waveform graph is the amplitude of (or, alternatively, power imparted through) the skin-contactable surface 106. The horizontal axis of each waveform graph represents time.

Figure 10A shows waveform 1000 in which controller 240 turns the power to motor 130 on and off at a fixed frequency or variable frequencies. When coupled with the appropriate skin-contactable element 105, waveform A may facilitate delivery of mechanical energy into an outer layer of skin (*i.e.,* the epidermis). This waveform may be suitable for medium to high intensity treatment and may be used, for example, with an amplitude of about 2mm and a frequency of about 1500-2000 cycles per minute.

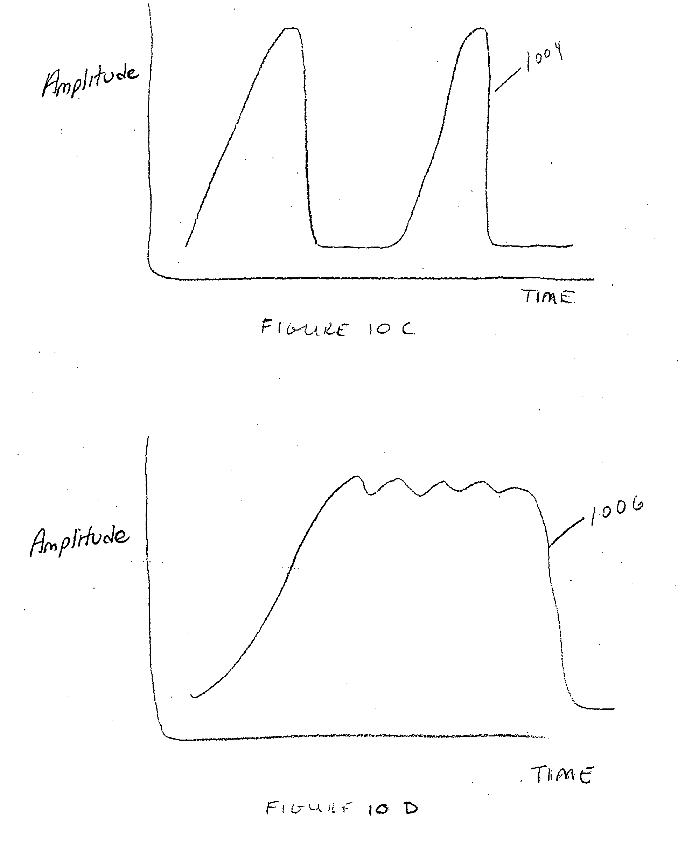

Similarly, Figure 10B shows waveform 1002 having a relatively long period spent at peak amplitude that may be suitable for high intensity treatment. Waveform 1002 may be used, for example, with an amplitude of about 2.5mm and a frequency of about 3000 cycles per minute. Figure 10C shows waveform 1004 having a relatively short period spent at peak amplitude that may be suitable for high intensity treatment that may be suitable for low intensity and may be used, for example, with an amplitude of about 1.5mm and a frequency of about 1000 cycles per minute.

Figure 10D depicts a high frequency waveform that may be superimposed on a lower frequency. The controller 240 gradually increases power to motor 130 until a plateau is reached. During the plateau period, oscillating power is provided to motor 130 at the high frequency, after which the power to motor 130 is gradually decreased. This waveform may be suitable for promoting energy delivery into outer layers of the skin 102 as well as into inner layers of the skin. Energy delivery to inner layers of the skin may serve to thus promote rejuvenation of collagen (from the high-frequency oscillations superimposed on the lower frequency).

### BENEFIT AGENTS

What is meant by a "benefit agent" is an element, an ion, a compound (*e.g*., a synthetic compound or a compound isolated from a natural source) or other chemical moiety in solid (*e.g*. particulate), liquid, or gaseous state and compound that has a cosmetic or therapeutic effect on the skin.

The compositions of the present invention may further include one or more benefit agents or pharmaceutically-acceptable salts and/or esters thereof, the benefit agents generally capable of interacting with the skin to provide a benefit thereto. As used herein, the term "benefit agent" includes any active ingredient that is to be delivered into and/or onto the skin at a desired location, such as a cosmetic or pharmaceutical.

The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed.

Examples of suitable benefit agents include those that provide benefits to the skin, such as, but not limited to, depigmentation agents; reflectants; film forming polymers; amino acids and their derivatives; antimicrobial agents; allergy inhibitors; anti-acne agents; anti-aging agents; anti-wrinkling agents, antiseptics; analgesics; shine-control agents; antipruritics; local anesthetics; anti-hair loss agents; hair growth promoting agents; hair growth inhibitor agents, antihistamines; antiinfectives; anti-inflammatory agents; anticholinergics; vasoconstrictors; vasodilators; wound healing promoters; peptides, polypeptides and proteins; deodorants and anti-perspirants; medicament agents; skin firming agents, vitamins; skin lightening agents; skin darkening agents; antifungals; depilating agents; counterirritants; hemorrhoidals; insecticides; enzymes for exfoliation or other functional benefits; enzyme inhibitors; poison ivy products; poison oak products; bum products; anti-diaper rash agents; prickly heat agents; vitamins; herbal extracts; vitamin A and its derivatives; flavenoids; sensates; anti-oxidants; hair lighteners; sunscreens; anti-edema agents, neo-collagen enhancers, film-forming polymers, chelating agents; anti-dandruff/sebhorreic dermatitis/psoriasis agents; keratolytics; and mixtures thereof.

In addition the benefit agent may also provide passive benefits to the skin. As such, the benefit agent may be formulated into a composition that include such ingredients as humectants or emollients, softeners or conditioners of the skin, make-up preparations, and mixtures thereof.

Examples of suitable anti-edema agents nonexclusively include bisabolol natural, synthetic bisabolol, corticosteroids, beta-glucans, and mixtures thereof.

Examples of suitable vasoconstrictors nonexclusively include horse chestnut extract, prickly ash, peroxides, tetrahydrozaline, and mixtures thereof.

Examples of suitable anti-inflammatory agents nonexclusively include benoxaprofen, centella asiatica, bisabolol, feverfew (whole), feverfew (parthenolide free), green tea extract, green tea concentrate, hydrogen peroxide, salicylates, oat oil, chamomile, and mixtures thereof.

Examples of neo-collagen enhancers nonexclusively include vitamin A and its derivatives (*e.g*. beta-carotene and retinoids such as retinoic acid, retinal, retinyl esters such as and retinyl palmitate, retinyl acetate and retinyl propionate); vitamin C and its derivatives such as ascorbic acid, ascorbyl phosphates, ascorbyl palmitate and ascorbyl glucoside; copper peptides; simple sugars such as lactose, mellibiose and fructose; and mixtures thereof.

Examples of enzymes include papain, bromelain, pepsin, and trypsin.

Examples of suitable skin firming agent nonexclusively include alkanolamines such as dimethylaminoethanol ("DMAE").

Examples of suitable antipruritics and skin protectants nonexclusively include oatmeal, beta-glucan, feverfew, soy products (by "soy product," it is meant a substance derived from soybeans, as described in United States Patent Application 2002-0160062), bicarbonate of soda, colloidal oatmeal, Anagallis Arvensis, Oenothera Biennis, Verbena Officinalis, and the like. As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size. Examples of suitable colloidal oatmeals include, but are not limited to, "Tech-O" available from the Beacon Corporation (Kenilworth, NJ) and colloidal oatmeals available from Quaker (Chicago, IL).

Examples of suitable reflectants nonexclusively include mica, alumina, calcium silicate, glycol dioleate, glycol distearate, silica, sodium magnesium fluorosilicate, and mixtures thereof.

Examples of skin darkening agents nonexclusively include dihydroxy acetone, erythulose, melanin, and mixtures thereof.

Suitable film forming polymers include those that, upon drying, produce a substantially continuous coating or film on the skin or nails. Nonexclusive examples of suitable film forming polymers include acrylamidopropyl trimonium chloride/acrylamide copolymer; corn starch/ acrylamide/ sodium acrylate copolymer; polyquatemium-10; polyquatemium-47; polyvinylmethylether/maleic anhydride copolymer; styrene/acrylates copolymers; and mixtures thereof.

Commercially available humectants which are capable of providing moisturization and conditioning properties nonexclusively include: (i) water soluble liquid polyols selected from the group comprising glycerine, propylene glycol, hexylene glycol, butylene glycol, pentylene glycol, dipropylene glycol, and mixtures thereof; (ii) polyalkylene glycol of the formula HO-(R"O)_{b}-H wherein R" is an alkylene group having from about 2 to about 4 carbon atoms and b is an integer of from about 1 to about 10, such as PEG 4; (iii) polyethylene glycol ether of methyl glucose of formula CH₃-C₆H₁₀O₅-(OCH₂CH₂)_{c}-OH wherein c is an integer from about 5 to about 25; (iv) urea; (v) fructose; (vi) glucose; (vii) honey; (viii) lactic acid; (ix) maltose; (x) sodium glucuronate; and (xi) mixtures thereof, with glycerine being an exemplary humectant.

Suitable amino acids and derivatives include amino acids derived from the hydrolysis of various proteins as well as the salts, esters, and acyl derivatives thereof. Examples of such amino acid agents nonexclusively include amphoteric amino acids such as alkylamido alkylamines, *i*.*e*. stearyl acetyl glutamate, capryloyl silk amino acid, capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; amino acids such as aspartic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline; lysine; silk amino acids, wheat amino acids; and mixtures thereof.

Suitable proteins include those polymers that have a long chain, *i.e*. at least about 10 carbon atoms, and a high molecular weight, *i.e*. at least about 1000, and are formed by self-condensation of amino acids. Nonexclusive examples of such proteins include collagen, deoxyribonuclease, iodized corn protein; milk protein; protease; serum protein; silk; sweet almond protein; wheat germ protein; wheat protein; alpha and beta helix of keratin proteins; hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Examples of suitable vitamins nonexclusively include various forms of vitamin B complex, including thiamine, nicotinic acid, biotin, pantothenic acid, choline, riboflavin, vitamin B3, vitamin B6, vitamin B12, pyridoxine, inositol, carnitine; vitamins A,C,D,E,K and their derivatives such as vitamin A palmitate and provitamins, *e.g*. (*i.e*., panthenol (pro vitamin B5) and panthenol triacetate) and mixtures thereof.

Examples of suitable antimicrobial agents nonexclusively include bacitracin, erythromycin, neomycin, tetracycline, chlortetracycline, benzethonium chloride, phenol, benzyl peroxide, metal salts or ions such as silver copper, zinc and their salts and mixtures thereof.

Examples of suitable skin emollients and skin moisturizers nonexclusively include mineral oil, lanolin, vegetable oils, isostearyl isostearate, glyceryl laurate, methyl gluceth-10, methyl gluceth-20 chitosan, and mixtures thereof.

An example of a suitable hair softener nonexclusively includes silicone compounds, such as those that are either non-volatile or volatile and those that are water soluble or water insoluble. Examples of suitable silicones include organo-substituted polysiloxanes, which are either linear or cyclic polymers of monomeric silicone/oxygen monomers and which nonexclusively include cetyl dimethicone; cetyl triethylammonium dimethicone copolyol phthalate; cyclomethicone; dimethicone copolyol; dimethicone copolyol lactate; hydrolyzed soy protein/dimethicone copolyol acetate; silicone quaternium 13; stearalkonium dimethicone copolyol phthalate; stearamidopropyl dimethicone; and mixtures thereof.

Examples of sunscreens, nonexclusively include benzophenones, bornelone, butyl paba, cinnamidopropyl trimethyl ammonium chloride, disodium distyrylbiphenyl disulfonate, PABA and its derivataives (such as octyl dimethyl PABA, butyl methoxydibenzoylmethane, isoamyl mnethoxycinnamate, methyl benzilidene camphor, octyl triazole, octyl methoxycinnamate, oxybenzone, octocrylene, octyl salicylate, homosalate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, Padimate O, red petrolatum, MEXORYL S and SX, TINOSORB M and S, and mixtures thereof.

Examples of skin lightening agents nonexclusively include hydroquinone, catechol and its derivatives, ascorbic acid and its derivatives, and mixtures thereof.

Examples of suitable insecticides (including insect repellents, anti-scabies and anti-lice treatments) nonexclusively include permethrin, pyrethrin , piperonyl butoxide, imidacloprid, N,N-diethyl toluamide, which refers to the material containing predominantly the *meta* isomer, *i.e*., N,N-diethyl-*m*-toluamide, which is also known as DEET; compounds of the formula III. 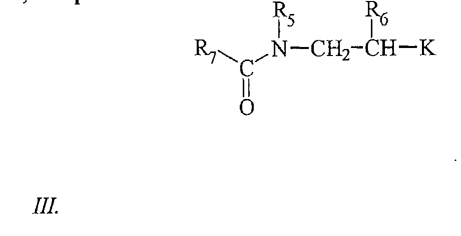 wherein
R₅ is a branched or unbranched alkyl group having about 1 to about 6 carbon atoms;
R₆ is H, methyl or ethyl;
R₇ is a branched or unbranched alkyl or alkoxy group having from about 1 to about 8 carbon atoms; and
K is a -CN or a -COOR₈ group, wherein
R₈ is a branched or unbranched alkyl group having from about 1 to about 6 carbon atoms,
natural or synthetic pyrethroids, whereby the natural pyrethroids are contained in pyrethrum, the extract of the ground flowers of *Chrysanthemum cinerariaefolium* or C *coccineum*; and mixtures thereof. Within the structure of Formula III. are ethyl 3-(N-butylacetamido)propionate, wherein R₇ is a CH₃ group, R₅ is an n-butyl group, R₆ is H, K is COOR₈ and R₈ is ethyl, which is available commercially from Merck KGaA of Darmstadt, Germany under the name, "Insect Repellent 3535."

Examples of an anti fungal for foot preparations nonexclusively includes tolnaftate and myconozole.

Examples of suitable depilating agents nonexclusively include calcium thioglycolate, magnesium thioglycolate, potassium thioglycolate, strontium thioglycolate, and mixtures thereof.

Examples of suitable analgesics such as external analgesics and local anesthetics nonexclusively include benzocaine, dibucaine, benzyl alcohol, camphor, capsaicin, capsicum, capsicum oleoresin, juniper tar, menthol, methyl nicotinate, methyl salicylate, phenol, resorcinol, turpentine oil, and mixtures thereof.

Examples of suitable antiperspirants and deodorants nonexclusively include aluminium chlorohydrates, aluminium zirconium chlorohydrates, and mixtures thereof.

Examples of suitable counterirritants nonexclusively include camphor, menthol, methyl salicylate, peppermint and clove oils, ichtammol, and mixtures thereof.

An example of a suitable inflammation inhibitor nonexclusively includes hydrocortisone, Fragaria Vesca, Matricaria Chamomilla, and Salvia Officinalis.

Examples of suitable anaesthetic ingredients nonexclusively include the benzocaine, pramoxine hydrochloride, lidocaine, betacaine and mixtures thereof; antiseptics such as benzethonium chloride; astringents such as zinc oxide, bismuth subgallate, balsam Peru, and mixtures thereof; skin protectants such as zinc oxide, silicone oils, petrolatum, cod liver oil, vegetable oil, and mixtures thereof.

Examples of such suitable benefits agents effective in the treatment of dandruff, seborrheic dermatitis, and psoriasis, as well as the symptoms associated therewith nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan ("elubiol"), clotrimazole, itraconazole, miconazole, climbazole, tioconazole, sulconazole, butoconazole, fluconazole, miconazole nitrate and any possible stereo isomers and derivatives thereof; piroctone olamine (Octopirox); ciclopirox olamine; anti-psoriasis agents such as vitamin D analogs, *e.g*. calcipotriol, calcitriol, and tacaleitrol; vitamin A analogs such as esters of vitamin A, *e.g*. vitamin A palmitate and vitamin A acetate, retinyl propionate, retinaldehyde, retinol, and retinoic acid; corticosteroids such as hydrocortisone, clobetasone, butyrate, clobetasol propionate menthol, pramoxine hydrochloride, and mixtures thereof.

Examples of benefit agents suitable for treating hair loss include, but are not limited to potassium channel openers or peripheral vasodilators such as minoxidil, diazoxide, and compounds such as N*-cyano-N-(tert-pentyl)-N'-3-pyridinyl-guanidine ("P-1075"); saw palmetto extract, vitamins, such as vitamin E and vitamin C, and derivatives thereof such as vitamin E acetate and vitamin C palmitate; hormones, such as erythropoietin, prostaglandins, such as prostaglandin El and prostaglandin F2-alpha; fatty acids, such as oleic acid; diruretics such as spironolactone; heat shock proteins ('HSP"), such as HSP 27 and HSP 72; calcium channel blockers, such as verapamil HCL, nifedipine, and diltiazemamiloride; immunosuppressant drugs, such as cyclosporin and Fk-506; 5 alpha-reductase inhibitors such as finasteride; growth factors such as, EGF, IGF and FGF; transforming growth factor beta; tumor necrosis factor; non-steroidal anti-inflammatory agents such as benoxaprofen; retinoids such as retinal and tretinoin; cytokines, such as IL-6, IL-1 alpha, and IL-1 beta; cell adhesion molecules such as ICAM; glucorcorticoids such as betametasone; botanical extracts such as aloe, clove, ginseng, rehmannia, swertia, sweet orange, zanthoxylum, Serenoa repens (saw palmetto), Hypoxis rooperi, stinging nettle, pumpkin seeds, and rye pollen; other botanical extracts including sandlewood, red beet root, chrysanthemum, rosemary, burdock root and other hair growth promoter activators; homeopathic agents such as Kalium Phosphoricum D2, Azadirachta indica D2, and Joborandi DI; genes for cytokines, growth factors, and male-pattered baldness; antifungals such as ketoconazole and elubiol; antibiotics such as streptomycin; proteins inhibitors such as cycloheximide; acetazolamide; benoxaprofen; cortisone; diltiazem; hexachlorobenzene; hydantoin; nifedipine; penicillamine; phenothaiazines; pinacidil; psoralens, verapamil; zidovudine; alpha-glucosylated rutin having at least one of the following rutins: quercetin, isoquercitrin, hespeddin, naringin, and methylhesperidin, and flavonoids and transglycosidated derivatives thereof; and mixtures thereof.

Examples of benefit agents suitable for use in inhibiting hair growth include: serine proteases such as trypsin; vitamins such as alpha-tocophenol (vitamin E) and derivatives thereof such as tocophenol acetate and tocophenol palmitate; antineoplastic agents, such as doxorubicin, cyclophosphamide, chlormethine, methotrexate, fluorouracil, vincristine, daunorubicin, bleomycin and hydroxycarbamide; anticoagulants, such as heparin, heparinoids, coumaerins, detran and indandiones; antithyroid drugs, such as iodine, thiouracils and carbimazole; lithium and lithium carbonate; interferons, such as interferon alpha, interferon alpha-2a and interferon alpha-2b; retinoids, such as retinol (vitamin A), isotretinoin: glucocorticoids such as betamethasone, and dexamethosone; antihyperlipidaemic drugs, such as triparanol and clofibrate; thallium; mercury; albendazole; allopurinol; amiodarone; amphetamines; androgens; bromocriptine; butyrophenones; carbamazcpine; cholestyramine; cimetidine; clofibrate; danazol; desipramine; dixyrazine; ethambutol; etionamide; fluoxetine; gentamicin, gold salts; hydantoins; ibuprofen; impramine; immunoglobulins; indandiones; indomethacin; intraconazole; levadopa; maprotiline; methysergide; metoprolol; metyrapone; nadolol; nicotinic acid; potassium thiocyanate; propranolol; pyridostimine; salicylates; sulfasalazine; terfenadine; thiamphenicol; thiouracils; trimethadione; troparanol; valproic acid; and mixtures thereof.

Examples of suitable anti-aging agents include, but are not limited to inorganic sunscreens such as titanium dioxide and zinc oxide; organic sunscreens such as octyl-methoxy cinnamates and derivatives thereof; retinoids; copper containing peptides; vitamins such as vitamin E, vitamin A, vitamin C, vitamin B, and derivatives thereof such as vitamin E acetate, vitamin C palmitate, and the like; antioxidants including beta carotene, alpha hydroxy acids such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, glycolic acid, isopropyl pyruvate, methyl pyruvate, mucic acid, pyruvic acid, saccharic acid, saccaric acid 1,4-lactone, tartaric acid, and tartronic acid; beta hydroxy acids such as beta-hydroxybutyric acid, beta-phenyl-lactic acid, beta-phenylpyruvic acid; polyphenolics; botanical extracts such as green tea, soy products, milk thistle, algae, aloe, angelica, bitter orange, coffee, goldthread, grapefruit, hoellen, honeysuckle, Job's tears, lithospermum, mulberry, peony, puerarua, nice, safflower, and mixtures thereof. Examples of suitable anti-acne agents include, but are not limited to topical retinoids (tretinoin, isotretinoin, motretinide, adapalene, tazarotene, azelaic acid, retinol); salicylic acid; benzoyl peroxide; resorcinol; antibiotics such as tetracycline and isomers thereof, erythromycin, and the anti-inflammatory agents such as ibuprofen, naproxen, hetprofen; botanical extracts such as alnus, arnica, artemisia capillaris, asiasarum root, birrh, calendula, chamomile, cnidium, comfrey, fennel, galla rhois, hawthorn, houttuynia, hypericum, jujube, kiwi, licorice, magnolia, olive, peppermint, philodendron, salvia, sasa albo-marginata; imidazoles such as ketoconazole and elubiol.

Examples of suitable depigmentation agents include, but are not limited to soy products, retinoids such as retinol; Kojic acid and its derivatives such as, for example, kojic dipalmitate; hydroquinone and it derivatives such as arbutin; transexamic acid; vitamins such as niacin, vitamin C and its derivatives; azelaic acid; placertia; licorice; extracts such as chamomile and green tea, and mixtures thereof, with retinoids, Kojic acid, soy products, and hydroquinone being particularly suitable examples.

Examples of suitable anti-hemorrhoidal products include, but are not limited to anesthetics such as benzocaine, pramoxine hydrochloride, and mixtures thereof; antiseptics such as benzethonium chloride; astringents such as zinc oxide, bismuth subgallate, balsam Peru, and mixtures thereof; skin protectants such as cod liver oil, vegetable oil, and mixtures thereof.

Examples of vasodilators include, but are not limited to minoxidil, diazoxide, and compounds such as N*-cyano-N-(tert-pentyl)-N'-3-pyridinyl-guanidine ("P-1075").

Examples of suitable shine-control agents include, but are not limited to hydrated silica, kaolin, bentonite. Examples of suitable anti-histamines include, but are not limited to diphenhydramine HCl. Examples of suitable antiinfectives include, but are not limited to benzalkonium chloride, hexamidine, and hydrogen peroxide. Examples of suitable wound healing promoters include, but are not limited to chitosan and its derivatives. Examples of suitable poison ivy and poison oak products include, but are not limited to bentonite, hydrocortisone, menthol, and lidocaine. Examples of bum products include, but are not limited to benzocaine and lidocaine. Examples of suitable anti-diaper rash products include, but are not limited to zinc oxide and petrolatum. Examples of suitable prickly heat products include, but are not limited to zinc oxide. Examples of suitable sensates include, but are not limited to menthol, fragrances, and capsaicin.

Benefit agents that may be particularly suitable for use with the apparatus 100 include, DMAE, soy products, colloidal oatmeal, sulfonated shale oil, olive leaf, elubiol, 6-(1-piperidinyl)-2,4-pyrimidinediamine-3-oxide, finasteride, ketoconazole, salicylic acid, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, copper containing compounds such as copper containing peptides and copper salts, hydrolyzed silk proteins, octyl methoxycinnamate, oxybenzone, avobenzone, minoxidil, saw palmetto extract, titanium dioxide, zinc dioxide, retinol, erthromycin, tretinoin, and mixtures thereof.

Benefit agents that may be of particularly suitable for use the apparatus 100 include neo-collagen promoters (*e.g*. retinoids such as retinal and copper-containing peptides), skin firming agents (*e.g*. DMAE), and depigmenting agents (*e.g*. soy).

The amount of the benefit agent that may be used may vary depending upon, for example, the ability of the benefit agent to penetrate through the skin or nail, the specific benefit agent chosen, the particular benefit desired, the sensitivity of the user to the benefit agent, the health condition, age, and skin and/or nail condition of the user, and the like. In sum, the benefit agent is used in a "safe and effective amount," which is an amount that is high enough to deliver a desired skin or nail benefit or to modify a certain condition to be treated, but is low enough to avoid serious side effects, at a reasonable risk to benefit ratio within the scope of sound medical judgment.

The benefit agent may be formulated, mixed, or compounded with other ingredients into a composition (*e.g*. liquid, emulsion, cream, and the like) wherein the other ingredients do not detract from the functionality of the benefit agent. A delivery agent that enhances the absorption of the one or more benefit agents into the skin may be formulated with the benefit agent to fulfill this function. Suitable delivery agents include, for example, sulfoxides, alcohols such as ethanol; fatty acids such as, for example, linoleic acid or oleic acid, fatty esters such as, for example, may be produced from reacting a C3-C10 carboxylic acid with a C10-C20 fatty alcohol; a polyol, an alkane, an amine, an amide, a turpene, a surfactant, a cyclodextrin or combinations thereof among other agents known to the art to be suitable for enhancing the penetration of various benefit agents through the stratum corneum into deeper layers of the skin.

The concentration of the benefit agent within the composition is variable. Unless otherwise expressed herein, typically the benefit agent is present in the composition in an amount, based upon the total weight of the composition/system, from about 0.01 percent to about 20 percent, such as from about 0.01 percent to about 5 percent (*e.g*., from about 0.01 percent to about 1 percent).

This composition that includes the benefit agent may also serve as a coupling composition as described previously and may include ingredients that enable the composition to possess one of these functions.

### DIAGNOSTIC SUB-SYSTEM

In one embodiment, a diagnostic sub-system 107 is used with the apparatus in the present invention. A diagnostic sub-system 107 is an electronic skin condition diagnostic system that uses the expanse of skin 102 to diagnose skin conditions by, for example, measuring mechanical properties such as skin elasticity; measuring chemical-mechanical properties such as water content and pH; or emitting light and detecting various wavelengths of radiation reflected or emitted by the expanse of skin 102. The diagnostic sub-system 107 may be used to image and/or otherwise characterize one or more properties or features of the skin 102, such as, but not limited to features or images associated with bumps, fine lines and wrinkles *e.g.,* crow's feet, other aspects of skin texture and surface roughness, scales, vellous hair, erythema (redness), blood vessel prominence and imaging, pigmentation such as pigmented macules, hyperpigmentation and the like, distribution of coproporphyrin produced by the bacteria *P. acnes*, among other skin features and properties.

In one embodiment of the invention, the diagnostic sub-system includes a small digital camera. The digital camera may be positioned within the shell 115 (shown in phantom in Figures 2A and 2B). For example, the digital camera may have an aperture spaced apart from the skin-contactable surface, through which an image can be collected and recorded onto a memory storage device associated with the digital camera, and also within the shell 115.

Further details of diagnostic system 105 and its elements are found in reference to U.S. Patent Application No. 20030086703 A1 and U.S. Patent Application No. 20030138249 A1. The diagnostic system 105 may communicate with the apparatus 100 via, for example, receiving element 255.

### METHOD OF USE

The following methods of using the apparatus 100 and the system 1 are consistent with embodiments of the invention described herein. Except where noted no specific order of steps is implied. The various method steps may be performed simultaneously or in various sequences to accomplish various results, such as treatment of the skin.

For embodiments of the invention in which the external diagnostic sub-system 107 is employed, the external diagnostic sub-system 107 is used to assess a state of one or more properties of the expanse of skin 102 of a subject.

A trained professional such as a professional dermatologist, researcher or technician may use results from the diagnostic sub-system 107 to produce treatment recommendations for the subject. The recommendations relate to tailoring the user output system 104 by selecting particular apparatus-enhancing agents, benefit agents, skin-contactable elements, amplitude, frequency and/or waveforms, or selection of the phase difference to be set between the multiple sub-surfaces 502, 504 (shown in Figures 5A-B); as well as possibly selecting a suitable sensing element 270. The diagnostic sub-system 107 may also help provide recommendations for any combination of these elements. Alternatively, the diagnostic sub-system 107 may be coupled to a database/expert system that is programmed to provide recommendations based upon information (e.g., images or other data) acquired by the diagnostic sub-system 107.

The recommendations that result from the use of the diagnostic sub-system 107 may be interrelated with one another in that a recommendation concerning one element may balance, complement or enhance another element as described below. For example, if the diagnostic sub-system 107 detects minimal acne, but a high concentration and severity of wrinkles, an aggressive or high intensity treatment may be desirable such as one that includes a high intensity waveform (*e*.*g*. waveform 1002, shown in Figure 10B with, for example, a 2.5 mm amplitude and 3000 cycles per minute). A skin-contactable surface 106 that is mild (*e.g*. one with smooth glass beads as shown in Figure 6A), thereby balancing the high intensity waveform. Alternatively, the recommendation may include an aggressive skin-contactable surface 106 (*e*.*g.* a skin-contactable surface with corundum or other harsh abrasive) to enhance rather than balance the effect high energy waveform.

As another example, if the diagnostic-sub system 107 detects redness, but no acne or wrinkles, a benefit agent such as retinol or hydroquinone may be chosen together with a mild waveform (such as waveform 1004, shown in Figure 10C, with an amplitude of 2mm and a frequency of 1000 cycles per minute) and a mild skin-contactable element 105 such as shown in Figure 6D, having recesses and no abrasive.

In another embodiment of the invention, such as, for example, if it is desirable to include a sensing element 270 that includes a thermal sensor to monitor surface temperature of the skin 102 (as may be desirable for treatments using a harsh abrasive on the skin contactable surface 106), a particular coupling composition that is thermally conductive (*e.g.*, one including alumina) may be recommended to complement the thermal sensor.

The above examples are meant to serve only as examples as to how the recommendation of one element may balance, complement or enhance another recommended element. Other attributes that may be considered in determining the one or more recommended elements include, but are not limited to: various properties of the one or more compositions or skin-contactable element 105 such as the ability to provide one or more benefits as described above in the section "BENEFIT AGENTS," analgesic properties, lubricity, surface activity or surface tension, abrasion, chemical compatibility, rate at which agent may be delivered to the skin 102, visco-elastic properties of the agent, exothermic/endothermic properties, among other properties.

In one embodiment, a pre-treatment composition is applied to the expanse of skin 102 prior to contacting the skin 102 with the skin-contactable surface 105. The pre-treatment composition is applied to the expanse of skin 102 prior to contacting the expanse of skin 102 with the skin-contactable surface 106. The pre-treatment composition may have coupling or homogenizing properties as discussed above in the section "APPARATUS-ENHANCING AGENTS." The pre-treatment composition may be applied via hand, or via chemical delivery sub-assembly 180.

In one embodiment of the invention, a skin treatment apparatus, such as, for example, the apparatus 100, is provided. The apparatus 100 is energized by, for example, plugging the apparatus into an external power source (if required) or merely selecting a switch on the keypad to provide power from the energy storage element 135.

A user turns on a switch (*e*.*g*., a component of input 260) to the on position, thereby providing power from energy storage element 135 to motor 130, causing transfer member 125 to, for example, move in one or more directions at fixed or variable frequencies. Periodic motion is transmitted from transfer member 125 to the skin-contactable surface 106. The user then contacts skin-contactable surface 106 with the expanse of the subject's skin. The skin-contactable surface 106 may then be moved (i.e., translated) across the expanse of skin to provide one or more skin benefits.

For embodiments of the invention in which the apparatus 100 includes a receiving element 255, a user may provide information to the apparatus by making selections on a keypad and/or inserting a computer readable medium into the standard port 250. The receiving element 255 thereby receives user-attribute data. The controller 240, coupled to the receiving element 255, receives data from the receiving element 255, and based upon the data provides action instructions to the user output assembly 104. For example, the user may program the controller 240 to provide particular current and/or voltage waveforms or delivery of particular compositions that customize the operation of skin-contactable surface 106 appropriate to the specific subject. Factors that the user may consider during the programming of controller 240 are, for example, the user's skin-type, age, body mass index, skin condition, global location, seasonal time of year, skin sensitivity, skin elasticity, and composition of the selected benefit agent. When controller 240 is programmed and the user is instructed in the use of apparatus 200, the user can provide his or her own treatment.

For example, if the user enters a skin-type that is classified as Type I (always bums), the controller 240 may select a high intensity waveform (e.g., waveform 1002) to provide more aggressive rejuvenating benefits. By contrast, if the user enters a skin type that is classified as skin-type IV (dark), the controller may select a mild waveform such as waveform 1004 as the required magnitude of rejuvenation may be relatively little. Similarly, if the user enters a skin-condition as "poor" (*e.g.*, a high concentration of wrinkles, for example), the relevant waveform provided by the controller 240 may be a high intensity one. This would contrast with an entry of a skin condition as "good," which the controller 240 may interpret as necessitating a low intensity waveform.

Similarly, the controller 240 may be programmed to intensify the waveform if the user inputs an age that is relatively high, a body-mass index (bmi) that is relatively high, a global location that is close to the equator, a seasonal time of year that is proximite to mid-summer, and/or a low degree of skin sensitivity.

The various inputs from the receiving element 255 such as skin type, age, bmi, and the like may be weighted equally by the controller or weighted unequally according to an algorithm.

In a manner similar to that described above for the receiving element 255, in embodiments of the invention in which the apparatus 100 includes the sensing element 270, data from the sensing element 270 is communicated to the controller 240 and may be processed alone or in conjunction with the information from the receiving element 255. Action instructions based upon this information may then be sent to the mechanical energy subassembly 112, the chemical delivery subassembly 180 or the indicator 245.

For example, the sensing element 270 may includes an ultraviolet (UV) light source and one or more photodiodes sensitive to UV light that may be reflected from the skin 102. If high levels of reflected ultraviolet light are detected from the expanse of skin 102, the controller may interpret this as a sign that acne lesions are present. As such, the amplitude and/or frequency of the waveform may be reduced (so as not to rupture the lesions) via a action instructions sent from the controller 240 to the motor 130.

In yet another embodiment of the invention, the sensing element 270 senses a pH or the presence or concentration of one or more chemical moieties such as products that may be produced during degradation of chemical moieties on the skin, and modifies the waveform as appropriate. Similarly, based upon information provided by the sensing element 270 a signal may be sent to the chemical delivery system 180 to select or change a dispense rate (including, for example, opening or closing valve 181 of the chemical delivery system 180) associated with of one or more of the compositions included therein.

For example, if the sensing element detects a pH that falls outside of the range of about 4.0 to about 5.5, valve 181 may open to allow a hyrdroxyacid treatment to be transported to the skin-contactable surface 106.

Referring again to Figure 9, according to one embodiment of the invention, various steps that may be performed by software governing the operation of the controller 240 in order to make use of information provided by the receiving element 255 and the sensing element 270 in order to effectuate appropriate output (*e*.*g*., waveforms or chemical delivery). The software may be stored in EPROM 1112 and microcontroller 1116. If there is external data to load into apparatus 100, the user loads data from external interface 255 to RAM 1110. The external data source may be a computer system, handheld computer device, or other data source connected to controller 240 via a USB port, RS-232 connection, or other means. The external data may be, for example, user-attribute data. Alternatively, the external data may be a set of instructions (data), such as may directly specify a waveform, a selection of benefit agent or coupling composition, or a time-dependent chemical delivery program to be executed via the controller 240.

The microcontroller 1116 monitors sensor input 1102 to determine whether there is sensor data provided from sensing element 270. If there is sensor data provided from sensor 270, A/D 1104 converts analog sensor data to digital format and microcontroller 1116 retrieves digital sensor data from sensor input 1102. Microcontroller 1116 modifies the waveform data loaded in RAM 1110 based on the sensor data retrieved. For example, if sensing element 270 indicates temperature data and the sensor data retrieved indicates that the temperature at skin-contactable element 105 has exceeded a prescribed limit, microcontroller 1116 may modify the waveform profile so that, upon waveform execution, the power delivered to motor 130 is, for example, reduced.

If there is no sensor data provided from sensing element 270, microcontroller 1116 retrieves, for example, the customized waveform data from RAM 1110 and instructs the elements of motor controller region 1118 to execute the waveform by delivering a corresponding current and voltage profile to motor 130. For example, microcontroller 1116 may instruct Is 1136 to ramp up current to delivered to motor 130 for a first time interval, deliver a fixed current at a certain frequency for a certain time interval, and ramp down current to a final current value.

If it is determined that there is no external data to load into apparatus 100, microcontroller 1116 determines whether there is more than one waveform profile to choose from in RAM 1110. If there is more than one waveform profile to choose from in RAM 1110, microcontroller 1116 utilizes indicator driver 1108 to prompt the user to select the appropriate waveform profile. In making a selection, the user operates input 260 and microcontroller 1116 instructs input controller 1106 to accept the data. The input data is communicated to microcontroller 1116, which selects the appropriate waveform profile from RAM 1110 that would be executed by program in EPROM 1112 to guide microcontroller 1116 to drive motor 130 and use data from indicator 245.

The method of treating the skin using mechanical energy may be continued for a time period until one of several events takes place. For example, the user may decide to end process based upon arbitrary factors, based upon a signal from the indicator 245 providing a signal to stop the process, based upon a signal from the signaling marker 810 in the skin-contactable element 105, based upon the controller 240 providing a signal to stop the motor 130 as may be pre-determined via software controlling the controller or based upon information provided from the sensing element 270 or the receiving element 255, among other events.

Upon ending the process of treating the skin with mechanical energy, a post-treatment composition (*e.g.*, a lubricious or moisturizing composition) and/or a benefit agent may be applied to the expanse of skin 102. The benefit agent may be applied as a post-treatment within a period of about 12 hours (such as preferably within about 30 minutes, such as within about 5 minutes) of completing the treatment with the apparatus 100.

Post-treatment with benefit agents is particularly beneficial in that benefits that may be imparted by the mechanical treatment of the apparatus are focused around exfoliation. By post-treating with benefit agents, in particular those benefit agents that operate by different mechanisms such as signal transduction, direct stimulus, and cellular modification, a faster onset and greater magnitude of benefits is possible that using mechanical treatment alone. Particularly suitable benefit agents for post-treatment include retinoids (*e*.*g*., retinol), copper moieties (*e*.*g*. copper-containing peptides), skin-filming agents (*e*.*g*. alkanolamines such as DMAE), and depigmenting agents (*e*.*g*., soy extracts).

It is to be understood that, while the invention has been described in conjunction with the detailed description thereof, the foregoing description is intended to illustrate and not to limit the scope of the invention. The invention is defined by the scope of the appended claims. Other aspects, advantages, and modifications are within the claims.

The following is a list of embodiments which are or may be claimed.
(A) A method of administering a skin benefit agent to an expanse of skin, said method comprising:
   (a) contacting said expanse of skin with a skin benefit agent; and
   (b) contacting said skin benefit agent on said expanse of skin with an apparatus having a output power to the skin of greater than about 0.2W, said apparatus comprising:
      (i) a skin-contactable element having a skin-contactable surface;
      (ii) a motor; and
      (iii) a transfer member for transferring mechanical energy from said motor to said skin-contactable element in order to provide periodic motion to said skin-contactable surface;
   wherein said skin-contactable surface contacts said skin-benefit agent.
(B) A method of administering a skin benefit agent to an expanse of skin, said method comprising:
   (a) contacting said expanse of skin with an apparatus, said apparatus comprising:
      (i) a skin-contactable element having a skin-contactable surface;
      (ii) a motor; and
      (iii) a transfer member for transferring mechanical energy from said motor to said skin-contactable element in order to provide periodic motion to said skin-contactable surface; and
   (b) after contacting said expanse of skin with said apparatus has ceased, further contacting said expanse of skin with a benefit agent, wherein said benefit agent is selected from the group consisting of retinoids, copper moieties, skin-firming agents, depigmentation agents, and combinations thereof.
(C) A product comprising a composition containing a skin benefit agent selected from the group consisting of retinoids, copper moieties, skin-firming agents, depigmentation agents, and combinations thereof, wherein said product further comprises instructions directing the user to apply said composition to an expanse of skin following contact of said expanse of skin with an apparatus that imparted mechanical energy to said expanse of skin.
(D) An apparatus for delivering mechanical energy to an expanse of skin, said apparatus comprising:
   (a) a skin-contactable element having a skin-contactable surface, wherein said skin-contactable element comprises an agent selected from the group consisting of a benefit agent, an apparatus-enhancing agent, and combinations thereof;
   (b) a motor; and
   (c) a transfer member for transferring mechanical energy from said motor to said skin-contactable element in order to provide periodic motion to said skin-contactable surface; wherein said agent is configured to release from the skin contactable element to said expanse of skin upon contacting said skin-contactable surface with said expanse of skin.
(E) A product comprising:
   (a) a skin-contactable element having a skin-contactable surface, wherein said skin-contactable element comprises an agent selected from the group consisting of a benefit agent, an apparatus-enhancing agent, and combinations thererof; and
   (b) instructions directing a user to couple the skin-contactable element to a motorized apparatus, wherein the motorized apparatus is for imparting mechanical energy to said skin-contactable surface;
   wherein said agent is configured to release from the skin contactable element to said expanse of skin upon contacting said skin-contactable surface with said expanse of skin.
(F) An apparatus for delivering mechanical energy to an expanse of skin, said apparatus comprising:
   (a) a skin-contactable element having a skin contactable surface;
   (b) a motor; and
   (c) a transfer member for transferring mechanical energy from said motor to said skin-contactable element in order to provide periodic motion to said skin-contactable surface;
   wherein the skin-contactable element comprises a signaling marker that is adapted to provide a change in a sensation that is discernable to a user after a period of time of operation of the apparatus, and wherein the sensation is selected from a group consisting of tactile, olfactory, thermal, visual, auditory, and combinations thereof.
(G) A product comprising:
   (a) a skin-contactable element comprising a signaling marker that is adapted to provide a change in a sensation that is discernable to a user after a period of time of operation of the apparatus, and wherein the sensation is selected from a group consisting of tactile, olfactory, thermal, visual, auditory, and combinations thereof; and
   (b) instructions directing a user to couple the skin-contactable element to a motorized apparatus, wherein the motorized apparatus is for imparting mechanical energy to the skin-contactable surface.
(H) An apparatus for delivering mechanical energy to skin, said apparatus comprising:
   (a) a user output assembly, the user output assembly comprising:
      (i) a skin-contactable element having a skin contactable surface;
      (ii) a motor; and
      (iii) a transfer member for transferring energy from said motor to said skin-contactable element a transfer member for transferring energy from said motor to said skin-contactable element;
   (b) a receiving element adapted to receive user-attribute data that is provided to the apparatus by a user; and
   (c) a controller coupled to the receiving element and said user output assembly, wherein the controller provides action instructions to the user output assembly based upon said user-attribute data received by said receiving element.
(I) An apparatus for delivering mechanical energy to an expanse of skin, said apparatus comprising:
   (a) a user output assembly, the user output assembly comprising:
      (i) a skin-contactable element having a skin contactable surface;
      (ii) a motor; and
      (iii) a transfer member for transferring energy from said motor to said skin-contactable element a transfer member for transferring energy from said motor to said skin-contactable element;
   (b) a sensing element in communication with the skin-contactable surface, wherein the sensing element is capable of sensing a state of at least one property associated with said expanse of skin, wherein said at least one property is selected from the group consisting of a thermal property, a chemical property, an optical property, and combinations thereof; and
   (c) a controller coupled to the sensing element and to said user output assembly, wherein said controller provides action instructions to the user output assembly based upon one or more of said at least one property sensed by said sensing element.
(J) The method of embodiment (A), wherein (i) said skin-contactable surface comprises a first sub-surface and a second subsurface and (ii) said method further comprises imparting a first periodic motion to said first sub-surface and a second periodic motion to said second subsurface such that said second subsurface moves in a disjoint relationship with respect to said first sub-surface.
(K) The method of embodiment (A), the apparatus of embodiment (D), or the product of embodiment (E) wherein said benefit agent is selected from a group consisting of reflectants ; film forming polymers; amino acids and their derivatives; allergy inhibitors; anti-acne agents; anti-aging agents; anti-wrinkling agents, antiseptics; analgesics; shine-control agents; antipruritics; ant-microbial agents; anti-hair loss agents; hair growth promoting agents; hair growth inhibitor agents, antihistamines; antiinfectives; anti-inflammatory agents; anticholinergics; vasoconstrictors; vasodilators; wound healing promoters; peptides, polypeptides and proteins; deodorants and anti-perspirants; film-forming polymers; vitamins; skin lightening agents; skin darkening agents; antifungals; depilating agents; counterirritants; enzymes and enzyme inhibitors; poison ivy products; poison oak products; bum products; anti-diaper rash agents; prickly heat agents; herbal extracts; flavenoids; sensates; anti-oxidants; hair lighteners ; chelating agents; keratolytics; sunscreens; anti-edema agents; and combinations thereof.
(L) The method of embodiment (A) wherein said skin-contactable element comprises said benefit agent and said benefit agent is transferred from said skin-contactable element to said expanse of skin.
(M) The method of embodiment (B) wherein said contacting begins after an interim period, wherein the interim period is defined by the time period between when the contacting of the expanse of skin with the skin-contactable surface has ceased and said contacting of the expanse of skin with the benefit agent has begun, wherein the interim period is less than about 12 hours.
(N) The method of embodiment (B) wherein said contacting begins after an interim period, wherein the interim period is defined by the time period between when the contacting of the expanse of skin with the skin-contactable surface has ceased and said contacting of the expanse of skin with the benefit agent has begun, wherein the interim period is less than about 30 minutes.
(O) The apparatus of embodiment (D) wherein said skin-contactable element further comprises a control layer forming at least a portion of said contactable surface, and wherein the control layer is configured to denature over the delay period.
(P) The product of embodiment (E) wherein said skin-contactable element further comprises a control layer forming at least a portion of the skin-contactable surface, wherein the control layer is configured to denature over the delay period.
(Q) A method for delivering mechanical energy to skin, the method comprising:
   (a) providing user-attribute data to said receiving element of the apparatus of embodiment (H); and
   (b) contacting an expanse of skin with a skin-contactable surface of said apparatus.
(R) A method for delivering mechanical energy to skin, said method comprising contacting an expanse of skin with a skin-contactable surface of the apparatus of embodiment (I).

## Claims

1. An apparatus for delivering mechanical energy to an expanse of skin, said apparatus comprising:
(a) a skin-contactable element having a skin-contactable surface comprising a first and second sub-surface, wherein said skin-contactable element comprises an agent selected from the group consisting of a benefit agent, an apparatus-enhancing agent, and combinations thereof;
(b) a motor; and
(c) a transfer member for transferring mechanical energy from said motor to said skin-contactable element in order to provide periodic motion to said skin-contactable surface;
wherein said periodic motion comprises a first periodic motion of said first sub-surface and a second periodic motion of said second subsurface such that said second subsurface moves in a disjoint relationship with respect to said first sub-surface.

2. The apparatus of claim 1, wherein said first periodic motion and said second periodic motion may be linear, rotational, or a combination thereof.

3. The apparatus of claim 3, wherein said phase difference may be set anywhere between 0 and 180 degrees.

4. The apparatus of any of the preceding claims, wherein a phase difference may be imparted between said first periodic motion and said second periodic motion.

5. The apparatus of claim 1 wherein the skin-contactable element comprises a signalling marker that is adapted to provide a change in a sensation that is discernable to a user after a period of time of operation of the apparatus, and wherein said sensation is selected from the group consisting of tactile, olfactory, thermal, visual, auditory, and combinations thereof.

6. The apparatus of claim 1 further comprising a receiving element adapted to receive user-attribute data that is provided to the apparatus by a user.

7. The apparatus of claim 6 further comprising a controller coupled to the receiving element and said apparatus, wherein the controller provides action instructions to the apparatus based upon said user-attribute data received by said skin-contactable element.

8. The apparatus of claim 7 further comprising a sensing element in communication with the skin-contactable surface, wherein the sensing element is capable of sensing a state of at least one property associated with said expanse of skin, wherein said at least one property is selected from the group consisting of a thermal property, a chemical property, an optical property, and combinations thereof.

9. The apparatus of claim 8 wherein the controller is coupled to the sensing element, wherein said controller provides action instructions based upon one or more of said at least one property sensed by said sensing element.

10. The apparatus of claim 1 wherein said benefit agent is selected from a group consisting of reflectants; film forming polymers; amino acids and their derivatives; allergy inhibitors; anti-acne agents; anti-aging agents; anti-wrinkling agents, antiseptics; analgesics; shine-control agents; antipruritics; ant-microbial agents; anti-hair loss agents; hair growth promoting agents; hair growth inhibitor agents, antihistamines; antiinfectives; anti-inflammatory agents; anticholinergics; vasoconstrictors; vasodilators; wound healing promoters; peptides, polypeptides and proteins; deodorants and anti-perspirants; film-forming polymers; vitamins; skin lightening agents; skin darkening agents; antifungals; depilating agents; counterirritants; enzymes and enzyme inhibitors; poison ivy products; poison oak products; bum products; anti-diaper rash agents; prickly heat agents; herbal extracts; flavenoids; sensates; anti-oxidants; hair lighteners; chelating agents; keratolytics; sunscreens; anti-edema agents; and combinations thereof.

11. The apparatus of claim 1 wherein said benefit agent is transferred from said skin-contactable element to said expanse of skin.
